# EUROPEAN PATENT APPLICATION

(11) **EP 1 238 982 A1**
(43) Date of publication of application: **11.09.2002**
(21) Application number: 02004353.5
(22) Date of filing: 04.03.2002
(51) Int. Cl.: C07K 14/435, C12N 15/62

(54) **A Fluorescent Protein**

(30) Priority: 05.03.2001 JP 2001059892
(71) Applicant: RIKEN, Wako-shi, Saitama 351-0198 (JP)
(72) Inventor: Miyawaki, Atsushi, c/o Riken, Wako-shi, Saitama 351-0198 (JP); Nagai, Takeharu, c/o Riken, Wako-shi, Saitama 351-0198 (JP)
(74) Representative: Sternagel, Fleischer, Godemeyer & Partner Patentanwälte

(57) **Abstract**

It is an object of the present invention to provide a novel fluorescent protein having sensitivity to a calcium ion, in particular a fluorescent protein which can be used as an indicator for quantifying calcium ion concentration. The present invention provides a fluorescent protein having the following amino acid sequences (1) to (3) in order in the direction from the N-terminus to the C-terminus, wherein a fused fluorescent protein obtained by fusion of the fluorescent protein with a calcium binding protein and its target peptide can emit fluorescence which is dependent on Ca²⁺ ion level;
(1) an amino acid sequence from the n^{th} amino acid from the N-terminus to the C-terminus of a fluorescent protein selected from the group consisting of a green fluorescent protein or its mutant, a yellow fluorescent protein or its mutant, a cyan fluorescent protein or its mutant, a red fluorescent protein or its mutant, and a blue fluorescent protein or its mutant, provided that n represents an integer of 140 to 150;
(2) a linker sequence of a sequence of 2 to 20 amino acids; and
(3) an amino acid sequence from the 1^{st} amino acid to the (n-1)^{th} amino acid from the N-terminus of the fluorescent protein described in (1) above.

## Description

### Technical Field

The present invention relates to a fluorescent protein which is useful for Ca²⁺ ion indicator. More particularly, the present invention relates to a fluorescent protein which is prepared by subjecting a fluorescent protein such as a green fluorescent protein or a yellow fluorescent protein to circular permutation, and can emit fluorescence which is dependent on Ca²⁺ ion level.

### Background Art

Application of green fluorescent protein (GFP)-based fluorescence resonance energy transfer (FRET) allows to visualize protein heteromerization and conformational changes in single living cells (Tsien, R Y. & Miyawaki, A. (1998) *Science* 280, 1954-1955). FRET utilizes two GFP mutants having different colors.

Wild-type GFP (WT-GFP) has a bimodal absorption spectrum with two peak maxima, at 395 and 475 nm, corresponding to the protonated and the deprotonated states of the chromophore, respectively (Tsien, R.Y. (1998) *Annu. Rev. Biochem.* 67, 509-44). The ionization state is modulated by a proton network, comprising an intricate network of polar interactions between the chromophore and several surrounding amino acids. In contrast to WT-GFP, the chromophore of most GFP variants titrates with single pKa values, indicating that the internal proton equilibrium has been distrupted as a result of an external manipulation (Llopis, J., McCaffery, J. M., Miyawaki, A., Farquhar, M. G & Tsien, R Y. (1998) *Proc. Natl. Acad. Sci.* USA 95, 6803-6808). One of the variants is the yellow fluorescent protein (YFP). It has a T203Y substitution which is responsible for the red-shift emission at 528 nm. It has been predicted that the tyrosine introduced at position 203 would be involved in a π-stacking interaction with the chromophore (Ormö, M., Cubitt, A. B., Kallio, K., Gross, L. A., Tsien, R Y. & Remington, S. J. (1996) *Science* 273, 1392-1395), which has been demonstrated by X-ray crystallography (Wachter, R M., Elsliger, M. A., Kallio, K., Hanson, G T. & Remington S. J. (1998) *Structure 6,* 1267-1277).

Within the rigid "β-can" structure (Yang, F., Moss, L. G & Phillips, G N., Jr (1996) *Nat*. Biotech. 14, 1246-1251) of GFP variants, Baird et al. found a site that would tolerate circular permutations where two portions of the polypeptide are flipped around the central site (Baird, G S., Zacharias, D. A. & Tsien, R Y (1999) *Proc. Natl. Acad. Sci.* USA 96, 11241-11246). With obvious clefts in the β-can, the chromophore of the circularly permuted GFPs (cpGFPs) seemed to be more accessible to protons outside of the proteins. The use of cpGFP might be interesting for converting the interaction signal between two protein domains into a change in the electrostatic potential of the chromophore, in other words, to transduce the information of the interaction into a fluorescent signal.

However, there has not been reported a cpGFP having sensitivity to a calcium ion, in particular a cpGFP which can be used as an indicator for quantifying calcium ion concentration.

### Summary of the Invention

It is an object of the present invention to provide a novel fluorescent protein having sensitivity to a calcium ion, in particular a fluorescent protein which can be used as an indicator for quantifying calcium ion concentration.

In order to achieve the above object, the present inventors have carried out concentrated research and have constructed cpYFP which was fused with calmodulin (CaM) and M13 (26 residue peptide derived from a CaM bonding region of skeletal muscle myosin light chain kinase) (Blumenthal, D.K., & Krebs, E.G (1987) Methods Enzymol. 139, 115-126). Ca²⁺-bound CaM (Ca²⁺-CaM) and M13 peptide form a stable and compact complex, and the solution structure of this complex has been measured by multidimensional NMR (Ikura, M., Clore, GM., Gronenborn A.M., Zhu, G, Klee, C.B. & Bax, A. (1992) Science 256, 632-638).

Further, the present inventors have studied the fluorescence characteristic of a chimeric fusion protein composed of M13, cpYFP and CaM, and have found that the fluorescence characteristic varies according to a Ca²⁺-dependent interaction between CaM and M13. In addition, it has been found that the behavior of spectral variation changes according to mutations which can influence the proton network of cpYFP. The present invention was completed on the basis of these findings.

Namely, according to the present invention, there is provided a fluorescent protein having the following amino acid sequences (1) to (3) in order in the direction from the N-terminus to the C-terminus, wherein a fused fluorescent protein obtained by fusion of the fluorescent protein with a calcium binding protein and its target peptide can emit fluorescence which is dependent on Ca²⁺ ion level;
(1) an amino acid sequence from the n^{th} amino acid from the N-terminus to the C-terminus of a fluorescent protein selected from the group consisting of a green fluorescent protein or its mutant, a yellow fluorescent protein or its mutant, a cyan fluorescent protein or its mutant, a red fluorescent protein or its mutant, and a blue fluorescent protein or its mutant, provided that n represents an integer of 140 to 150;
(2) a linker sequence of a sequence of 2 to 20 amino acids; and
(3) an amino acid sequence from the 1^{st} amino acid to the (n-1)^{th} amino acid from the N-terminus of the fluorescent protein described in (1) above.

Preferably in the present invention, under the presence of Ca²⁺ ions, the fused fluorescent protein can emit fluorescence of an intensity which differs depending on Ca²⁺ ion level, or under the presence of Ca²⁺ ions, the fused fluorescent protein can show excitation of a wavelength which differs depending on Ca²⁺ ion level.

Preferably, the amino acid sequence of a linker sequence is Gly-Gly-Ser-Gly-Gly or Val-Asp-Gly-Gly-Ser-Gly-Gly-Thr-Gly.

Examples of the fluorescent protein of the present invention include any of the followings;
(A) a protein having the amino acids from position 32 to 275 of the amino acid sequence shown in SEQ ID NO:1, or a protein having an amino acid sequence having a deletion, substitution and/or addition of one to several amino acids in the amino acids from position 32 to 275 of the amino acid sequence shown in SEQ ID NO:1 and having a fluorescence characteristic that is equivalent to or greater than that of a protein having the amino acids from position 32 to 275 of the amino acid sequence shown in SEQ ID NO:1;
(B) a protein having the amino acids from position 32 to 278 of the amino acid sequence shown in SEQ ID NO:2, or a protein having an amino acid sequence having a deletion, substitution and/or addition of one to several amino acids in the amino acids from position 32 to 278 of the amino acid sequence shown in SEQ ID NO:2 and having a fluorescence characteristic that is equivalent to or greater than that of a protein having the amino acids from position 32 to 278 of the amino acid sequence shown in SEQ ID NO:2; or,
(C) a protein having the amino acids from position 32 to 278 of the amino acid sequence shown in SEQ ID NO:3, or a protein having an amino acid sequence having a deletion, substitution and/or addition of one to several amino acids in the amino acids from position 32 to 278 of the amino acid sequence shown in SEQ ID NO:3 and having a fluorescence characteristic that is equivalent to or greater than that of a protein having the amino acids from position 32 to 278 of the amino acid sequence shown in SEQ ID NO:3.

According to another aspect of the present invention, there is provided a fused fluorescent protein having the following amino acid sequences (1) to (5) in order in the direction from the N-terminus to the C-terminus, which can emit fluorescence that is dependent on Ca²⁺ ion level.
(1) an amino acid sequence of a target peptide of a calcium-binding protein;
(2) an amino acid sequence from the n^{th} amino acid from the N-terminus to the C-terminus of a fluorescent protein selected from the group consisting of a green fluorescent protein or its mutant, a yellow fluorescent protein or its mutant, a cyan fluorescent protein or its mutant, a red fluorescent protein or its mutant, and a blue fluorescent protein or its mutant, provided that n represents an integer of 140 to 150;
(3) a linker sequence of a sequence of 2 to 20 amino acids;
(4) an amino acid sequence from the 1^{st} amino acid to the (n-1)^{th} amino acid from the N-terminus of the fluorescent protein described in (2) above; and
(5) the amino acid sequence of a calcium-binding protein.

Preferably, the fused fluorescent protein of the present invention can, under the presence of Ca²⁺ ions, emit fluorescence of an intensity that differs depending on Ca²⁺ ion level, or can, under the presence of Ca²⁺ ions, show excitation of a wavelength that differs depending on Ca²⁺ ion level.

Preferably, the amino acid sequence of a linker sequence is Gly-Gly-Ser-Gly-Gly or Val-Asp-Gly-Gly-Ser-Gly-Gly-Thr-Gly.

Preferably, the calcium-binding protein is a protein selected from the group consisting of: calmodulin, troponin C, calcineurin B, myosin light chain, recoverin, S-modulin, visinin, VILIP, neurocalcin, hippocalcin, frequenin, caltractin, calpain large-subunit, S100 proteins, parvalbumin, calbindin D_{9K}, calbindin D_{28K}, and calretinin.

Examples of the fused fluorescent protein of the present invention include any of the followings;
(A) a protein having the amino acid sequence shown in SEQ ID NO:1, or a protein having an amino acid sequence having a deletion, substitution and/or addition of one to several amino acids in the amino acid sequence shown in SEQ ID NO:1 and having a fluorescence characteristic that is equivalent to or greater than that of a protein having the amino acid sequence shown in SEQ ID NO:1;
(B) a protein having the amino acid sequence shown in SEQ ID NO:2, or a protein having an amino acid sequence having a deletion, substitution and/or addition of one to several amino acids in the amino acid sequence shown in SEQ ID NO:2 and having a fluorescence characteristic that is equivalent to or greater than that of a protein having the amino acid sequence shown in SEQ ID NO:2; or,
(C) a protein having the amino acid sequence shown in SEQ ID NO:3, or a protein having an amino acid sequence having a deletion, substitution and/or addition of one to several amino acids in the amino acid sequence shown in SEQ ID NO:3 and having a fluorescence characteristic that is equivalent to or greater than that of a protein having the amino acid sequence shown in SEQ ID NO:3.

According to still another aspect of the present invention, there is provided a calcium ion indicator which comprises the aforementioned fused fluorescent protein, as well as a method for measuring concentration or distribution of intracellular calcium ion by using the aforementioned fused fluorescent protein.

According to still another aspect of the present invention, there is provided DNA encoding the aforementioned fluorescent protein. Examples thereof include any of the following DNA;
(A) DNA having the nucleotide sequence from position 94 to 825 of the nucleotide sequence shown in SEQ ID NO:1, or DNA having a nucleotide sequence having a deletion, substitution and/or addition of one to several nucleotides in the nucleotide sequence from position 94 to 825 of the nucleotide sequence shown in SEQ ID NO:1 and encoding a protein having a fluorescence characteristic that is equivalent to or greater than that of a protein encoded by DNA having the nucleotide sequence from position 94 to 825 of the nucleotide sequence shown in SEQ ID NO:1;
(B) DNA having the nucleotide sequence from position 94 to 834 of the nucleotide sequence shown in SEQ ID NO:2, or DNA having a nucleotide sequence having a deletion, substitution and/or addition of one to several nucleotides in the nucleotide sequence from position 94 to 834 of the nucleotide sequence shown in SEQ ID NO:2 and encoding a protein having a fluorescence characteristic that is equivalent to or greater than that of a protein encoded by DNA having the nucleotide sequence from position 94 to 834 of the nucleotide sequence shown in SEQ ID NO:2; or,
(C) DNA having the nucleotide sequence from position 94 to 834 of the nucleotide sequence shown in SEQ ID NO:3, or DNA having a nucleotide sequence having a deletion, substitution and/or addition of one to several nucleotides in the nucleotide sequence from position 94 to 834 of the nucleotide sequence shown in SEQ ID NO:3 and encoding a protein having a fluorescence characteristic that is equivalent to or greater than that of a protein encoded by DNA having the nucleotide sequence from position 94 to 834 of the nucleotide sequence shown in SEQ ID NO:3.

According to still another aspect of the present invention, there is provided DNA encoding the aforementioned fused fluorescent protein. Examples thereof include any of the following DNA:
(A) DNA having the nucleotide sequence shown in SEQ ID NO:1, or DNA having a nucleotide sequence having a deletion, substitution and/or addition of one to several nucleotides in the nucleotide sequence shown in SEQ ID NO:1 and encoding a protein having a fluorescence characteristic that is equivalent to or greater than that of a protein encoded by the nucleotide sequence shown in SEQ ID NO:1;
(B) DNA having the nucleotide sequence shown in SEQ ID NO:2, or DNA having a nucleotide sequence having a deletion, substitution and/or addition of one to several nucleotides in the nucleotide sequence shown in SEQ ID NO:2 and encoding a protein having a fluorescence characteristic that is equivalent to or greater than that of a protein encoded by the nucleotide sequence shown in SEQ ID NO:2; or
(C) DNA having the nucleotide sequence shown in SEQ ID NO:3, or DNA having a nucleotide sequence having a deletion, substitution and/or addition of one to several nucleotides in the nucleotide sequence shown in SEQ ID NO:3 and encoding a protein having a fluorescence characteristic that is equivalent to or greater than that of a protein encoded by the nucleotide sequence shown in SEQ ID NO:3.

According to still another aspect of the present invention, there is provided a recombinant vector having the aforementioned DNA.

According to still another aspect of the present invention, there is provided a transformant having the aforementioned DNA or the aforementioned recombinant vector.

According to still another aspect of the present invention, there is provided a method for measuring the calcium ion concentration or distribution in the aforementioned transformant by using the fluorescence emitted by the transformant as an index.

According to still another aspect of the present invention, there is provided a kit for measuring calcium ions which comprises at least one or more selected from the fluorescent protein, the fused fluorescent protein, the calcium ion indicator, the DNA, the recombinant vector, or the transformant, each of which are mentioned above.

### Brief Description of the Drawings

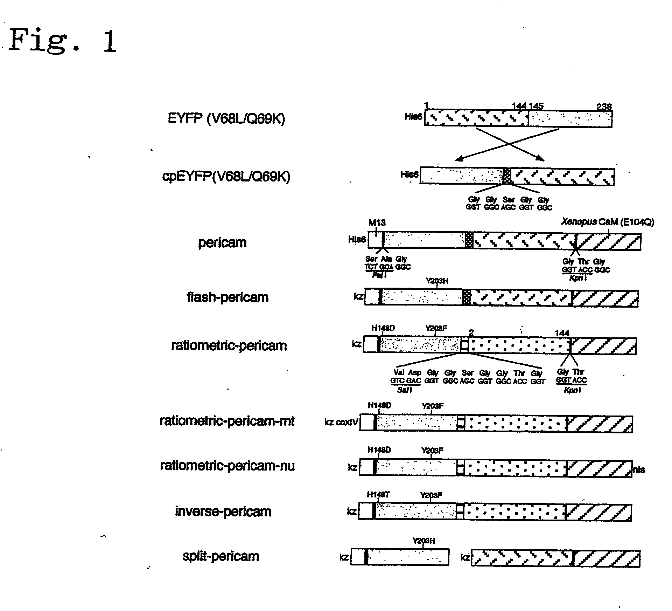

Figure 1 shows a schematic structures and sequences of pericams for expression in bacteria and mammalian cells. Sequences of linkers and amino acid substitutions are shown below and above the bars, respectively. His6, the polyhistidine tag; kz, Kozak consensus sequence; nls, nuclear localization signal; coxIV, cytochrome *c* oxidase subunit IV targeting signal (Rizzuto, R, Simpson, A. W., Brini, M & Pozzan, T. (1992) *Nature* 358, 325-327).

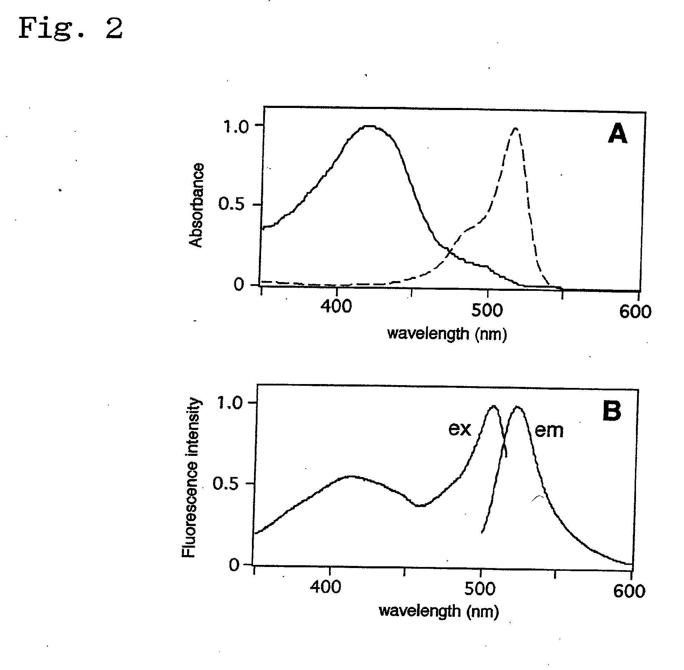

Figure 2(A) shows an absorbance spectra of EYFP(V68L/Q69K) (broken line) and cpEYFP(V68L/Q69K) (solid line), and Figure 2(B) shows a fluorescence excitation (ex) and emission (em) spectra of cpEYFP(V68L/Q69K), recorded at 530-nm emission and 485-nm excitation, respectively. All spectra were normalized to a maximum value of 1.0.

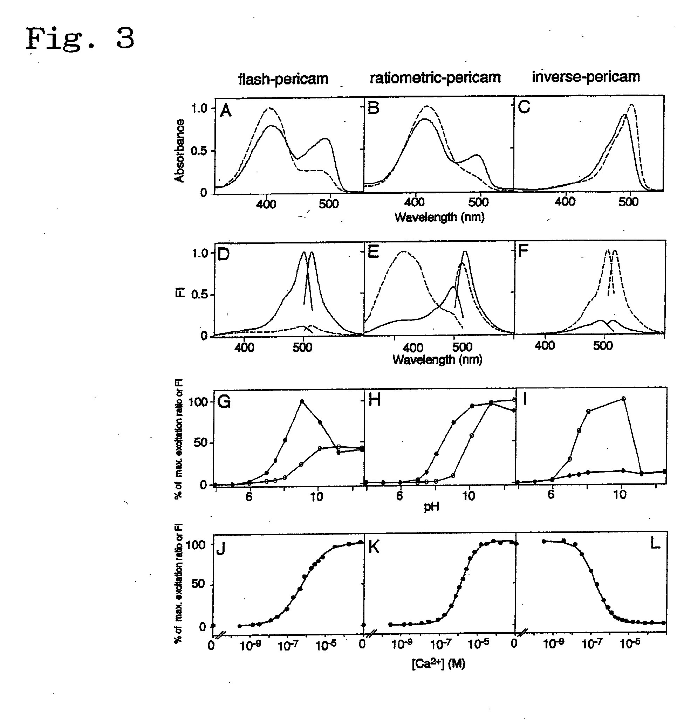

Figure 3 shows an *In vitro* properties of flash-pericam (A, D, G and J), ratiometric-pericam (B, E, H and K), and inverse-pericam (C, F, I and L). Figure3 shows absorbance (A to C) and fluorescence excitation and emission (D to F) spectra of pericams, pH-dependency of normalized amplitudes in 514-nm emission peak (G), 516-nm emission peak (I), and excitation ratio of 495/410 (H). In (A) to (I), the spectra and data points were obtained in the presence (solid line) and absence (broken line) of Ca²⁺ ion. In (J) to (L), Ca²⁺ titration curves of pericams are shown. FI means fluorescence intensity.

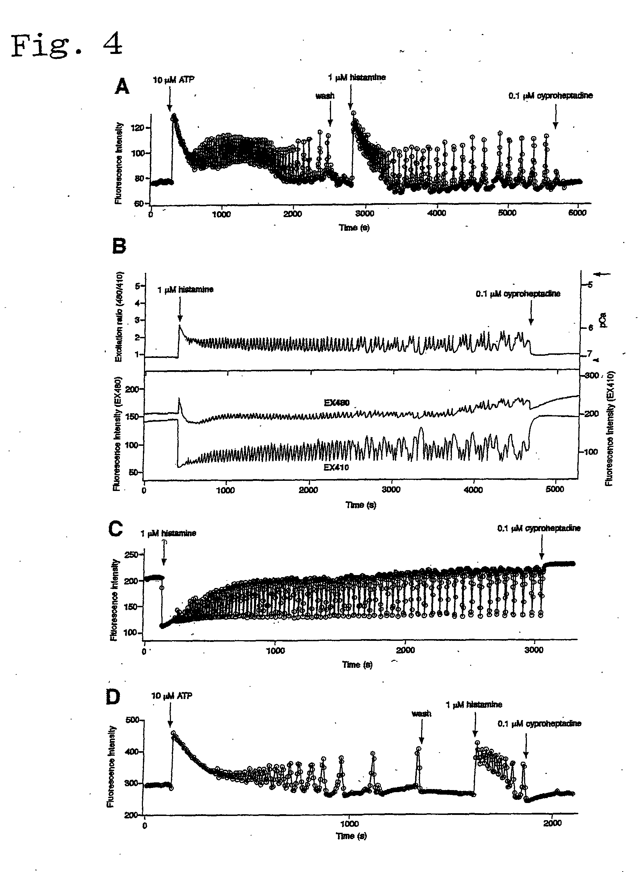

Figure 4 shows typical [Ca²⁺]ᵢ transients and oscillations induced by receptor-stimulations in HeLa cells expressing flash-pericam (A), ratiometric-pericam (B), inverse-pericam (C) and split-pericam (D). The sampling interval was 3 to 5 seconds. (B) Top, 480 to 410 nm excitation ratios. The right-hand ordinate calibrates [Ca²⁺]ᵢ in µM with *R*ₘₐₓ and *R*ₘᵢₙ indicated by an arrow and arrowhead, respectively. Bottom, 480 nm (black line, left-hand scale) and 410 nm (gray line, right-hand scale) excitations.

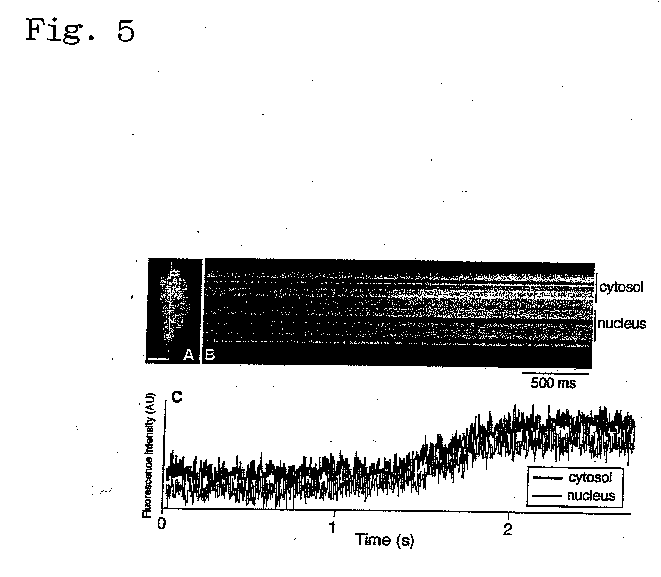

Figure 5(A) shows a confocal image of a HeLa cell expressing flash-pericam; Figure 5(B) shows a line-scanned image along a longitudinal line in (A) of the histamine-evoked Ca²⁺ propagation; and Figure (C) shows the time-course of Ca²⁺ signals in the cytosol and nucleus; their areas are indicated on the right side of (B). Scale bar, 10 µm.

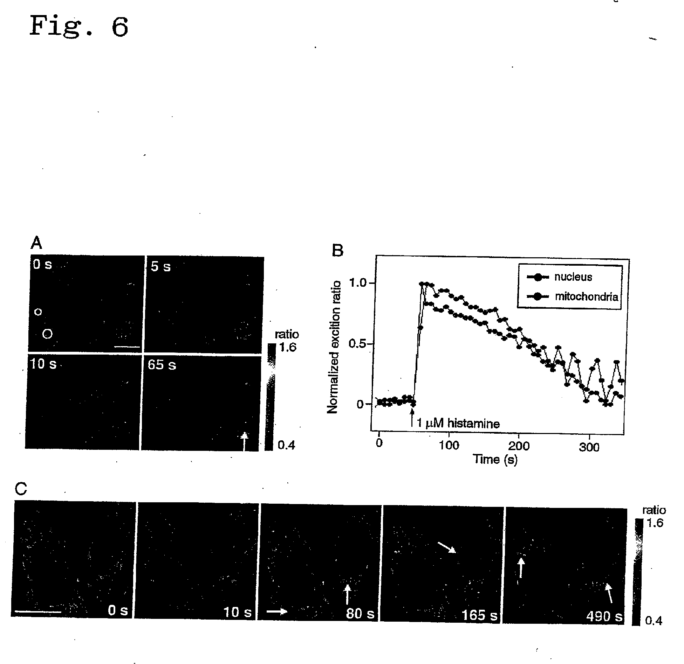

Figure 6(A) shows a series of pseudocolored images of 4 HeLa cells that expressed ratiometric-pericam-nu and -mt. From the left top to the right bottom, 0, 5, 10 and 65 seconds after 1 µM histamine application. A small and a large circles in the leftmost cell are regions of interest for measurements of [Ca²⁺]ₙ and [Ca²⁺]ₘ, respectively. Their time-courses are shown in (B) with indication of the time points at which the 4 images of (A) were acquired. Figure 6(C) shows a series of pseudocolored images of 2 HeLa cells expressing ratiometric-pericam-mt. Arrows indicate subpopulations of mitochondria transiently exhibiting high [Ca²⁺]ₘ. Scale bar, 10 µm.

### MODE FOR CARRYING OUT THE INVENTION

The embodiments and methods for carrying out the present invention are described in detail below.

### (1) A fluorescent protein of the present invention

The present invention relates to a fluorescent protein having the following amino acid sequences (1) to (3) in order in the direction from the N-terminus to the C-terminus, wherein a fused fluorescent protein obtained by fusion of the fluorescent protein with a calcium binding protein and its target peptide can emit fluorescence which is dependent on Ca²⁺ ion level;
(1) an amino acid sequence from the n^{th} amino acid from the N-terminus to the C-terminus of a fluorescent protein selected from the group consisting of a green fluorescent protein or its mutant, a yellow fluorescent protein or its mutant, a cyan fluorescent protein or its mutant, a red fluorescent protein or its mutant, and a blue fluorescent protein or its mutant, provided that n represents an integer of 140 to 150;
(2) a linker sequence of a sequence of 2 to 20 amino acids; and
(3) an amino acid sequence from the 1^{st} amino acid to the (n-1)^{th} amino acid from the N-terminus of the fluorescent protein described in (1) above.

The term "a green fluorescent protein or its mutant; a yellow fluorescent protein or its mutant; a cyan fluorescent protein or its mutant; a red fluorescent protein or its mutant; and a blue fluorescent protein or its mutant" used in the present specification has a meaning to include all known fluorescent proteins or their mutants. For example, GFP gene has been isolated and its sequence has also been determined (Prasher, D.C. et al.(1992), "Primary structure of the Aequorea victoria green fluorescent protein", Gene 111:229-233). There have also been numerous reports of amino acid sequences of other fluorescent proteins or their mutants, for example, as described in Roger Y. Tsin, Annu. Rev. Biochem. 1998. 67:509-44 and the literature cited therein. In Delagrave, S. et al. (1995), Red-shifted excitation mutants of the green fluorescent protein, Bio/Technology 13:151-154, there has been isolated a cloned Aequorea victoria GFP mutant having a red-shifted excitation spectrum. In Heim, R et al. (1994), Wavelength mutations and posttranslational autoxidation of green fluorescent protein, Proc. Natl. Acad. Sci. USA 91:12501-12504, there is reported a mutant (His from Tyr₆₆) having blue fluorescence represented by BFP(Tyr₆₆→His).

The fluorescent protein of the present invention adopts a structure having; the amino acid sequence from the n^{th} amino acid from the N-terminus to the C-terminus of the above-described fluorescent protein on the N-terminus side (n represents an integer from 140 to 150); the amino acid sequence from the 1^{st} amino acid of the N-terminus to the n-1 amino acid of the above-described fluorescent protein on the C-terminus side; and a linker sequence sandwiched between these two amino acid sequences. Altering the structure of a protein in this manner is also referred to as "circular permutation." In the present invention, by applying circular permutation to known fluorescent proteins such as those described above, production of a novel fluorescent protein which is useful as a calcium ion indicator has been succeeded for the first time.

The amino acid sequence of the linker sequence is not particularly limited as long as the produced fused fluorescent protein exerts the desired effect as a calcium ion indicator. It is preferable that the amino acid sequences comprises mainly relatively small side-chains. Also, amino acids having a hydrophilic side-chains are preferred. The number of amino acids is normally between 2-20, preferably 3-10, and more preferably 5-10. Examples of a linker sequence include Gly-Gly-Ser-Gly-Gly (SEQ ID NO:4) or Val-Asp-Gly-Gly-Ser-Gly-Gly-Thr-Gly (SEQ ID NO:5), although the linker sequence is not limited thereto.

Examples of a fluorescent protein of the present invention include any of the following fluorescent proteins.
(A) a protein having the amino acids from position 32 to 275 of the amino acid sequence shown in SEQ ID NO:1, or a protein having an amino acid sequence having a deletion, substitution and/or addition of one to several amino acids in the amino acids from position 32 to 275 of the amino acid sequence shown in SEQ ID NO:1 and having a fluorescence characteristic that is equivalent to or greater than that of a protein having the amino acids from position 32 to 275 of the amino acid sequence shown in SEQ ID NO:1;
(B) a protein having the amino acids from position 32 to 278 of the amino acid sequence shown in SEQ ID NO:2, or a protein having an amino acid sequence having a deletion, substitution and/or addition of one to several amino acids in the amino acids from position 32 to 278 of the amino acid sequence shown in SEQ ID NO:2 and having a fluorescence characteristic that is equivalent to or greater than that of a protein having the amino acids from position 32 to 278 of the amino acid sequence shown in SEQ ID NO:2; or,
(C) a protein having the amino acids from position 32 to 278 of the amino acid sequence shown in SEQ ID NO:3, or a protein having an amino acid sequence having a deletion, substitution and/or addition of one to several amino acids in the amino acids from position 32 to 278 of the amino acid sequence shown in SEQ ID NO:3 and having a fluorescence characteristic that is equivalent to or greater than that of a protein having the amino acids from position 32 to 278 of the amino acid sequence shown in SEQ ID NO:3.

In the term "an amino acid sequence having a deletion, substitution and/or addition of one to several amino acids" used in the present specification, the range of "one to several" is not particularly limited, but is, for example, from 1 to 20, preferably 1 to 10, more preferably 1 to 7, still more preferably 1 to 5, and particularly preferably 1 to 3.

The term "having a fluorescence characteristic that is equivalent to or greater than" is used herein to mean that, the characteristic of fluorescence emitted dependent on Ca²⁺ ion level by a fused fluorescent protein obtained by fusing a calcium binding protein and its target peptide to the fluorescent protein, is equivalent or greater.

The method for obtaining a fluorescent protein of the present invention is not particularly limited. There may be used any of a chemically synthesized protein, or a recombinant protein produced by a gene recombination technique.

In the case of producing a recombinant protein, it is necessary to obtain the DNA encoding the protein. By utilizing the information of the amino acid sequence and the nucleotide sequence shown in SEQ ID NOS:1-3 of the sequence list herein, suitable primers can be designed, and by using those to perform PCR using a cDNA clone of various known fluorescent proteins as described above as a template, DNA fragments necessary to construct DNA encoding a fluorescent protein of the present invention can be prepared. Next, by ligating those DNA fragments in order by means of a gene recombination technique, DNA encoding the desired fluorescent protein can be obtained. A fluorescent protein of the present invention can then be produced by introducing this DNA into a suitable expression system. Expression in the expression system is described later in the present specification.

### (2) A fused fluorescent protein of the present invention and a use thereof.

The present invention further relates to a fused fluorescent protein having the amino acid sequence of a target peptide of a calcium-binding protein at the N-terminus of a fluorescent protein described in (1) above, and the amino acid sequence of a calcium-binding protein at the C-terminus.

Examples of a calcium-binding protein used in the present invention include calmodulin, troponin C, calcineurin B, myosin light chain, recoverin, S-modulin, visinin, VILIP, neurocalcin, hippocalcin, frequenin, caltractin, calpain large-subunit, S100 proteins, parvalbumin, calbindin D_{9K}, calbindin D_{28K}, and calretinin. Among these, in particular, the use of calmodulin is preferred.

The amino acid sequence of a target peptide of a calcium-binding protein present on the N-terminus side of a fused fluorescent protein of the present invention can be suitably selected by a person skilled in the art depending on the calcium-binding protein present on the C-terminus side.

For example, in the case where the calcium-binding protein is calmodulin, an amino acid sequence of a calmodulin-binding domain, which is known to exist in various proteins and peptides known to be target substances of calmodulin, can be present at the N-terminus side of a fused fluorescent protein of the present invention. Currently, over 1,200 types of amino acid sequence of calmodulin-binding domain are known, and it is possible to search for these on a calmodulin binding domain database (http://calcium.oci.utoronto.ca/ctdb).

Examples of a fused fluorescent protein of the present invention include any of the following fused fluorescent proteins.
(A) a protein having the amino acid sequence shown in SEQ ID NO:1, or a protein having an amino acid sequence having a deletion, substitution and/or addition of one to several amino acids in the amino acid sequence shown in SEQ ID NO:1 and having a fluorescence characteristic that is equivalent to or greater than that of a protein having the amino acid sequence shown in SEQ ID NO:1;
(B) a protein having the amino acid sequence shown in SEQ ID NO:2, or a protein having an amino acid sequence having a deletion, substitution and/or addition of one to several amino acids in the amino acid sequence shown in SEQ ID NO:2 and having a fluorescence characteristic that is equivalent to or greater than that of a protein having the amino acid sequence shown in SEQ ID NO:2; or,
(C) a protein having the amino acid sequence shown in SEQ ID NO:3, or a protein having an amino acid sequence having a deletion, substitution and/or addition of one to several amino acids in the amino acid sequence shown in SEQ ID NO:3 and having a fluorescence characteristic that is equivalent to or greater than that of a protein having the amino acid sequence shown in SEQ ID NO:3.

The method for obtaining a fused fluorescent protein of the present invention is not particularly limited, and there may be used a chemically synthesized protein, or a recombinant protein produced by a gene recombination technique.

In the case of producing a recombinant protein, it is necessary to obtain the DNA encoding the protein. By utilizing the information of the amino acid sequence and the nucleotide sequence shown in SEQ ID NOS:1-3 of the sequence list herein, suitable primers can be designed. By using those primers to perform PCR using a cDNA clone of various known fluorescent proteins as described above as a template, it is possible to produce the DNA fragments necessary to construct DNA encoding a fluorescent protein. Also, by means of a known technique such as PCR using suitable primers, DNA fragments are produced that encode a calcium-binding protein and a target peptide of the calcium-binding protein, respectively. Next, by connecting these DNA fragments in order by a gene recombination technique, DNA encoding the desired fused fluorescent protein can be obtained. By introducing this DNA into a suitable expression system, a fused fluorescent protein of the present invention can be produced. The expression in the expression system is described later in the present specification.

A fused fluorescent protein of the present invention can be used for measuring intracellular calcium ion concentration or for monitoring calcium ion distribution, and is useful as a calcium ion indicator. Calcium ion concentration and calcium ion distribution can be monitored by introducing a gene encoding a fused fluorescent protein of the present invention into a cell. Measurement of fluorescence can be conducted according to a known method.

### (3) DNA of the present invention

The present invention also provides a gene encoding a fluorescent protein or fused fluorescent protein of the present invention.

Examples of DNA encoding a fluorescent protein of the present invention include any of the following DNA.
(A) DNA having the nucleotide sequence from position 94 to 825 of the nucleotide sequence shown in SEQ ID NO:1, or DNA having a nucleotide sequence having a deletion, substitution and/or addition of one to several nucleotides in the nucleotide sequence from position 94 to 825 of the nucleotide sequence shown in SEQ ID NO:1 and encoding a protein having a fluorescence characteristic that is equivalent to or greater than that of a protein encoded by DNA having the nucleotide sequence from position 94 to 825 of the nucleotide sequence shown in SEQ ID NO:1;
(B) DNA having the nucleotide sequence from position 94 to 834 of the nucleotide sequence shown in SEQ ID NO:2, or DNA having a nucleotide sequence having a deletion, substitution and/or addition of one to several nucleotides in the nucleotide sequence from position 94 to 834 of the nucleotide sequence shown in SEQ ID NO:2 and encoding a protein having a fluorescence characteristic that is equivalent to or greater than that of a protein encoded by DNA having the nucleotide sequence from position 94 to 834 of the nucleotide sequence shown in SEQ ID NO:2; or,
(C) DNA having the nucleotide sequence from position 94 to 834 of the nucleotide sequence shown in SEQ ID NO:3, or DNA having a nucleotide sequence having a deletion, substitution and/or addition of one to several nucleotides in the nucleotide sequence from position 94 to 834 of the nucleotide sequence shown in SEQ ID NO:3 and encoding a protein having a fluorescence characteristic that is equivalent to or greater than that of a protein encoded by DNA having the nucleotide sequence from position 94 to 834 of the nucleotide sequence shown in SEQ ID NO:3.

Further, examples of DNA encoding a fused fluorescent protein of the present invention include any of the following DNA.
(A) DNA having the nucleotide sequence shown in SEQ ID NO:1, or DNA having a nucleotide sequence having a deletion, substitution and/or addition of one to several nucleotides in the nucleotide sequence shown in SEQ ID NO:1 and encoding a protein having a fluorescence characteristic that is equivalent to or greater than that of a protein encoded by the nucleotide sequence shown in SEQ ID NO:1;
(B) DNA having the nucleotide sequence shown in SEQ ID NO:2, or DNA having a nucleotide sequence having a deletion, substitution and/or addition of one to several nucleotides in the nucleotide sequence shown in SEQ ID NO:2 and encoding a protein having a fluorescence characteristic that is equivalent to or greater than that of a protein encoded by the nucleotide sequence shown in SEQ ID NO:2; or
(C) DNA having the nucleotide sequence shown in SEQ ID NO:3, or DNA having a nucleotide sequence having a deletion, substitution and/or addition of one to several nucleotides in the nucleotide sequence shown in SEQ ID NO:3 and encoding a protein having a fluorescence characteristic that is equivalent to or greater than that of a protein encoded by the nucleotide sequence shown in SEQ ID NO:3.

In the term "a nucleotide sequence having a deletion, substitution and/or addition of one to several nucleotides" used in the present specification, the range of "one to several" is not particularly limited, but is, for example, from 1 to 60, preferably 1 to 30, more preferably 1 to 20, still more preferably 1 to 10, and particularly preferably 1 to 5.

The DNA of the present invention or its fragments can, for example, be synthesized by the phosphoramidite method or the like, and can be produced by polymerase chain reaction (PCR) using specific primers. The method of preparation of the DNA of the present specification or its fragments is as described herein in (1) A fluorescent protein of the present invention, and (2) A fused fluorescent protein of the present invention and a use thereof

Further, a method for introducing a desired mutation into certain nucleotide sequence is known to persons skilled in the art. For example, it is possible to construct DNA having a mutation by suitably using a known technique such as site-directed mutagenesis, PCR using a degenerate oligonucleotide, exposure of a cell which contains nucleic acid to a mutagenetic agent or radiation, or the like. Such known techniques are described in, for example, Molecular Cloning: A laboratory Manual, 2^{nd} Ed., Cold Spring Harbor Laboratory, Cold Spring Harbor, N.Y,1989, and Current Protocols in Molecular Biology, Supplement 1-38, John Wiley & Sons (1987-1997).

### (4) The recombinant vector of the present invention

The DNA of the present invention can be used after insertion into a suitable vector. The types of vectors used in the present invention are not particularly limited. For example, the vector may be a vector which replicates autonomously (for example, a plasmid), or may be a vector which is integrated into the genome of the host cell when introduced into the host cell and is replicated along with the chromosome.

Preferably, the vector used in the present invention is an expression vector. In an expression vector, elements necessary for transcription (for example, a promoter and the like) are operatively linked to the DNA of the present invention. A promoter is a DNA sequence which exhibits transcription activity in a host cell, and the promoter can be suitably selected in accordance with the type of host.

Examples of promoters which are operative in bacterial cells include, a promoter of Bacillusstearothermophilus maltogenic amylase gene, Bacillus licheniformis alpha-amylase gene, Bacillus amyloliquefaciens BAN amylase gene, Bacillus subtilis alkaline protease gene, or Bacillus pumilus xylosldase gene; a P_{R} or P_{L} promoter of phage lambda; a lac, trp, or tac promoter of *Escherichia coli;* and the like.

Examples of promoters which are operative in mammalian cells include SV40 promoter, MT-1 (metallothionein gene) promoter, adenovirus 2 major late promoter, and the like. Examples of promoters which are operative in insect cells include polyhedrin promoter, P10 promoter, basic protein promoter of Autographa californica nuclear polyhedrosis, baculovirus immediate early gene 1 promoter, baculovirus 39K delayed early gene promoter, and the like. Examples of promoters which are operative in yeast host cells include a promoter derived from yeast glycolysis system genes, alcohol dehydrogenase gene promoter, TPI1 promoter, ADH2-4c promoter, and the like.

Examples of promoters which are operative in mycotic cells include ADH3 promoter, tpiA promoter, and the like.

Further, if necessary, the DNA of the present invention may be operatively linked to a suitable terminator, such as a human growth hormone terminator, or TPI1 terminator or ADH3 terminator for a fungal host. The recombinant vector of the present invention may also have an element such as a polyadenylation signal (for example, one derived from SV40 or the adenovirus 5E1b region), a transcription enhancer sequence (for example, SV40 enhancer), or a translation enhancer sequence (for example, one encoding adenovirus VA RNA).

The recombinant vector of the present invention may also be provided with a DNA sequence which enables the vector to replicate inside the host cell, and an example thereof is an SV40 origin of replication (when the host cell is a mammalian cell).

The recombinant vector of the present invention may also contain a selectable marker. Examples of a selectable marker include genes whose complement is absent in the host cell such as dihydrofolate reductase (DHFR) or schizosaccharomyces pombe TPI gene, or drug resistance genes such as ampicillin, kanamycin, tetracycline, chloramphenicol, neomycin, and hygromycin.

A method for connecting the DNA of the present invention, a promoter, and, as desired, a terminator and/or a secretion signal sequence, respectively, and inserting these into a suitable vector is known to persons skilled in the art.

### (5) A transformant of the present invention

A transformant can be produced by introducing the DNA or recombinant vector of the present invention into a suitable host.

A host cell into which the DNA or recombinant vector of the present invention is introduced may be any cell which can express the DNA construct of the present invention, and examples include bacteria, yeast, fungi, and higher eukaryotic cells.

Examples of bacterial cells include Gram-positive bacteria such as Bacillus and Streptomyces, and Gram-negative bacteria such as *Escherichia coli.* Transformation of these bacteria may be performed by the protoplast method or a known method using competent cells.

Examples of mammalian cells include HEK293 cell, HeLa cells, COS cell, BHK cell, CHL cell, and CHO cell. Methods for transforming a mammalian cell and expressing the DNA sequence inserted into the cell are known. For example, methods such as electroporation, phosphate calcium method, or lipofection can be used.

Examples of yeast cells include cells belonging to Saccharomyces or Schizosaccharomyces, and examples include *Saccharomyces cerevisae* and *Saccharomyces kluyveri*. Examples of methods for introducing a recombinant vector to a yeast host include electroporation, the spheroplast method, and the lithium acetate method.

Other examples of fungal cells are filamentous bacteria, for example Aspergillus, Neurospora, and Fusarium, and cells belonging to Trichoderma. When a filamentous bacterium is used as a host cell, transformation can be performed by integrating the DNA construct into the host chromosome and obtaining a recombinant host cell. Integration of the DNA construct into the host chromosome can be performed according to a known method such as homologous recombination or heterologous recombination.

When an insect cell is used as a host, a protein can be expressed by co-transfecting a recombinant gene transduction vector and a baculovirus into the insect cell to obtain a recombinant virus in the insect cell culture supernatant, and then infecting the recombinant virus into the insect cell (for example, as described in Baculovirus Expression Vectors, A Laboratory Manual, and Current Protocols in Molecular Biology, Bio/Technology, 6, 47 (1988), and the like).

As a baculovirus, for example, Autographa californica nuclear polyhedrosis virus, which is a virus that infects Mamestra-family insects, and the like, can be used.

As an insect cell, for example, Sf9 and Sf21, which are ovarian cells of *Spodoptera frugiperda* [Baculovirus Expression Vectors, A Laboratory Manual, W. H. Freeman and Company, New York, (1992)], and Hi Five, which is an ovarian cell of *Trichoplusia ni* (Invitrogen), or the like can be used.

Examples of a method for co-transfection of a recombinant gene transduction vector and the above-described baculovirus into an insect cell for producing a recombinant virus, include phosphate calcium method or lipofection.

The above-described transformant is cultured in a suitable nutrient culture medium under conditions which enable expression of the introduced DNA construct. To isolate and purify a fluorescent fused protein of the present invention from the culture of the transformant, a conventional method for isolating and purifying a protein may be used.

For example, in a case where a protein of the present invention was expressed in a solubilized form within cells, after culturing is completed, cells are recovered by centrifugation and suspended in a water-based buffer, then a cell-free extract is obtained by disrupting the cells by means of an ultrasonic homogenizer or the like. From the supernatant obtained by centrifuging the cell-free extract, a purified sample can be obtained using a conventional method for isolating and purifying a protein. Examples of the conventional method for isolating and purifying a protein include the use of, either alone or in combination, techniques such as solvent extraction, salting-out by ammonium sulfate or the like, desalting, precipitation by an organic solvent, anion exchange chromatography using a resin such as Diethylaminoethyl (DEAE) Sepharose, cation exchange chromatography using a resin such as S-Sepharose (Pharmacia), hydrophobic chromatography using a resin such as Butyl Sepharose or Phenyl Sepharose, gel filtration using a molecular sieve, affinity chromatography, chromatofocusing, an electrophoresis method such as isoelectric focusing, and the like.

### (6) Method for measuring concentration or distribution of intracellular calcium ion

In the method of the present invention, by measuring a cell transformed or transfected by a DNA construct of the present invention by means of a spectrophotometer or a fluorescence microscope, the spectral characteristics of a cell in a liquid culture can be determined. Thus, the calcium ion concentration or distribution in the transformant can be measured.

Specifically, by introducing a DNA construct comprising DNA encoding a fused fluorescent protein of the present invention into a cell, monitoring is enabled. Methods for introducing DNA into a cell, culturing a cell, and detecting a fluorescent protein are known to persons skilled in the art.

A simple method for detecting a fluorescent protein of the present invention is the use of a fluorometer, that is, a device equipped with a light source for exciting a fluorescent protein of the present invention and photometer for measuring fluorescence intensity. A fluorometer is a well-known device, and is commercially available. In addition, calcium ion distribution can also be determined by imaging a fluorescence using a fluorescence microscope.

### (7) Kit of the present invention

According to the present invention, there is provided a kit for measuring calcium ions, which comprises at least one or more which are selected from a fluorescent protein, a fused fluorescent protein, a calcium ion indicator comprising the protein, DNA, a recombinant vector or a transformant, each of which are described in the present specification. The kit of the present invention can be prepared by known materials and techniques which are conventionally used in the art.

The reagent such as the fluorescent protein and DNA can be prepared in a form suitable for preservation by dissolving it in a suitable solvent. Examples of a suitable solvent include water, ethanol, various buffer solutions, and the like.

The present invention is illustrated by the following Examples, but the present invention is not limited by the Examples.

### Examples

### (A) Materials and Methods

### (A-1) Gene construction

The cDNA of cpEYFP(V68L/Q69K) was constructed by three separate PCRs. First, the cDNA of the 3' portion of EYFP(V68L/Q69K) (Miyawaki, A., Griesbeck, O., Heim, R & Tsien, R Y. (1999) *Proc. Natl. Acad. Sci. USA* 96, 2135-2140) was amplified with a sense primer containing a *Pst*I site, and a reverse primer encoding a peptide linker GGSGG Second, the cDNA of the 5' portion of EYFP(V68L/Q69K) was amplified with a sequence encoding the peptide linker GGSGG and a *Kpn*I site to the 5' and 3' ends, respectively. Finally, the entire cDNA of cpEYFP(V68L/Q69K) was amplified with the *Pst*I-and *Kpn*I-site containing primers using a mixture of the first and second PCR fragments as the template. The restricted product was cloned in-frame into the *Pst*I/*Kpn*I sites of pRSET_{B} (Invitrogen), yielding cpEYFP(V68L/Q69K)/pRSET_{B}. Then the cDNAs encoding M13 and CaM were amplified using primers containing 5' *Bam*HI and 3' *Pst*I sites, and 5' *Kpn*I and 3' *Eco*RI sites, respectively. The restricted PCR fragments were ligated to the 5' and 3' ends of cpEYFP(V68L/Q69K) gene in pRSET_{B} to yield the construct of pericam for bacterial expression. The modification of the linkers and site-directed mutagenesis at 148 and 203 in cpEYFP(V68L/Q69K) were performed as previously described (Sawano, A. & Miyawaki, A. (2000) *Nucleic Acids Res.* 28, e78), to create flash-(SEQ ID NO.1), ratiometric-(SEQ ID NO.2) and inverse-(SEQ ID NO.3) pericams. The 5' end of the pericam gene was modified by PCR to have a *Hin*dIII site followed by a Kozak consensus sequence (CCACCATG). The *Hind*III/*Eco*RI fragment encoding the pericam was subcloned in the mammalian expression vector pcDNA3 (Invitrogen). The cDNAs coding for M13-EYFP(V68L/Q69K)(145-238) and EYFP(V68L/Q69K)(1-144)-CaM were obtained from flash-pericam cDNA and cloned into pcDNA3. Ratiometric-pericam-mt and ratiometric-pericam-nu were obtained by extending the ratiometric-pericam cDNA at the 5' end with the sequence encoding the N-terminal 12 amino acid presequence of subunit IV of cytochrome *c* oxidase (Rizzuto, R, Simpson, A. W., Brini, M & Pozzan, T. (1992) *Nature* 358, 325-327), and at the 3' end with the nuclear localization signal: PKKKRKVEDA, respectively.

### (A-2) Protein expression and in vitro spectroscopy

Recombinant fluorescent proteins with polyhistidine tag at N-terminus were expressed in Escherichia coli (JM109(DE3)), purified and spectroscopically characterized as previously described (Miyawaki, A., Llopis, J., Heim, R, McCaffery, J. M., Adams, J. A., Ikura, M. & Tsien, R Y. (1997) *Nature* 388, 882-887).

### (A-3) pH- and Ca²⁺ - titrations

pH titrations were performed using a series of buffers prepared with pHs ranging from 4 to 12.5 as previously described (Ormö, M., Cubitt, A. B., Kallio, K., Gross, L. A., Tsien, R. Y. & Remington, S. J. (1996) *Science* 273, 1392-1395). Ca²⁺ titrations were performed by reciprocal dilution of Ca²⁺-free and Ca²⁺-saturated buffers prepared by using O,O'-Bis(2-aminoethyl)ethyleneglycol-N,N,N',N'-tetraacetic acid (EGTA), N-(2-hydroxyethyl)ethylenediamine-N,N',N'-triacetic acid (EDTA-OH) or Nitrilotriacetic acid (NTA).

### (A-4) Mammalian expression and Imaging

Two or three days after cDNA transfection with Superfect (QIAGEN), HeLa cells in HBSS buffer were imaged at 25°C on an Olympus IX-70 with a MicroMax-1300Y/HS interlined CCD camera (Roper scientific Inc.), controlled by MetaFluor/MetaMorph 4.0 software (Universal imaging). Single-wavelength imaging with flash-pericam, inverse-pericam and split-pericam used a 475DF35 excitation filter, a 505 DRLP dichroic mirror, and an HQ525/50 emission filter. Dual-excitation imaging with ratiometric-pericam used two excitation filters (480DF10 and 410DF10) which were alternated by a filter changer (Lambda 10-2, Sutter Instruments), a 505 DRLP-XR dichroic mirror, and a 535DF25 emission filter. Confocal images were carried out using a Fluoview FV500 confocal laser scanning microscope (Olympus) with an argon-ion laser (Omnichrome, Melles Griot).

### (B) Results and Discussions

### (B-1) Development of Ca²⁺-sensitive circularly permuted GFP variants

A YFP variant, EYFP(V68L/Q69K) (Miyawaki, A., Griesbeck, O., Heim, R & Tsien, R Y. (1999) *Proc. Natl. Acad. Sci. USA* 96, 2135-2140) was subjected to circular permutation. The original N- and C- termini were linked through a pentapeptide linker GGSGG, rendering Y145 and N144 new N- and C-termini, respectively (Figure 1). With a hexa-histidine tag (His6-tag) attached at the N-terminus, the circularly permuted YFP (cpEYFP(V68L/Q69K)) was expressed in *E.coli* and purified using the His6-tag. Spectra of purified proteins were all measured at pH 7.4 unless otherwise stated. The absorbance spectrum of cpEYFP(V68L/Q69K) shows a major peak at 420 nm with a small shoulder near 500 nm (Figure 2A), contrasting with the original EYFP(V68L/Q69K) peaking predominantly at 514 nm (Figure 2A, Miyawaki, A., Griesbeck, O., Heim, R & Tsien, R Y. (1999) *Proc. Natl. Acad. Sci. USA* 96, 2135-2140). This blue-shift suggests that the chromophore was protonated in cpEYFP(V68L/Q69K). The excitation spectrum had two peaks at 417 and 506 nm (Figure 2B), which was reminiscent of the bimodal excitation spectrum of WT-GFP (Tsien, R. Y. (1998) *Annu. Rev. Biochem.* 67, 509-44). The protonated species of cpEYFP(V68L/Q69K) absorbing around 420 nm fluoresced, while protonated species of most YFPs do not (Tsien, R Y. (1998) *Annu. Rev. Biochem.* 67, 509-44).

Our initial attempt was to connect the C-terminus of CaM and N-terminus of M13 with the N- and C-termini of cpEYFP(V68L/Q69K), respectively. However, the resulting chimera protein did not show any response to Ca²⁺. Then, CaM and M13 were interchanged: cpEYFP(V68L/Q69K) was fused to the C-terminus of M13 through a tripeptide linker SAG, and through a GTG linker to the N-terminus of CaM¹³ containing the E104Q mutation of the conserved bidentate glutamate in the third Ca²⁺ binding loop to glutamine (Figure 1). Although the N-terminus of CaM and the C-terminus of M13 are 50 Å apart in their complex (Ikura, M., Clore, G. M., Gronenborn A. M., Zhu, G, Klee, C. B. & Bax, A. (1992) *Science* 256, 632-638), the chimeric protein was fluorescent and showed Ca²⁺ sensitivity. The protein having a circularly permuted YFP and a CaM, was named "pericam". When excited at 485 nm, the Ca²⁺-bound pericam showed an emission peak at 520 nm, 3 times brighter than the Ca²⁺-free pericam. On the contrary, neither absorbance nor fluorescence spectrum of cpEYFP(V68L/Q69K) was sensitive to Ca²⁺.

To obtain pericams with a larger dynamic range, several amino acids involved in the proton network were optimized. Substitution of His203 for Tyr improved the dynamic range significantly. The new pericam, called "flash-pericam" (Figure 1), exhibited an 8-times increase in the fluorescence with Ca²⁺ (Figure 3D). Together with two mutations which provide a better folding at 37°C (V163A and S175G), flash-pericam was designed to work as a single wavelength indicator of intracellular Ca²⁺. In the absence of Ca²⁺, flash-pericam exhibited similar absorbance spectrum to that of cpEYFP(V68L/Q69K) (Figure 3A, broken line). Upon saturation with Ca²⁺, the 490-nm absorbance peak increased at the expense of the 400-nm peak (Figure 3A, solid line), indicating that association of the Ca²⁺-CaM with M13 peptide caused ionization of the chromophore, which resulted in a leftward shift of the pH-titration curve (Figure 3G). It should be noted that when the pH was high enough to ionize the chromophore, the Ca²⁺-bound flash-pericam (at pH = 9) was about twice as bright as the Ca²⁺-free one (at pH>10). Besides the pH-titrability, therefore, the interaction between CaM and M13 might have direct steric effects on the chromophore, and change its ionization state or reduce its out-of-plane distortions that could result in enhanced radiationless decay. The letter possibility is likely, because Ca²⁺ binding to flash-pericam increased the quantum yield by several times as well as the molar extinction coefficient around 490 nm (Table 1). The pH titration curve in Figure 3G also shows that the Ca²⁺-bound flash-pericam was alkaline-quenched (pH>10), suggesting the collapse of such an incomplete β-can structure.

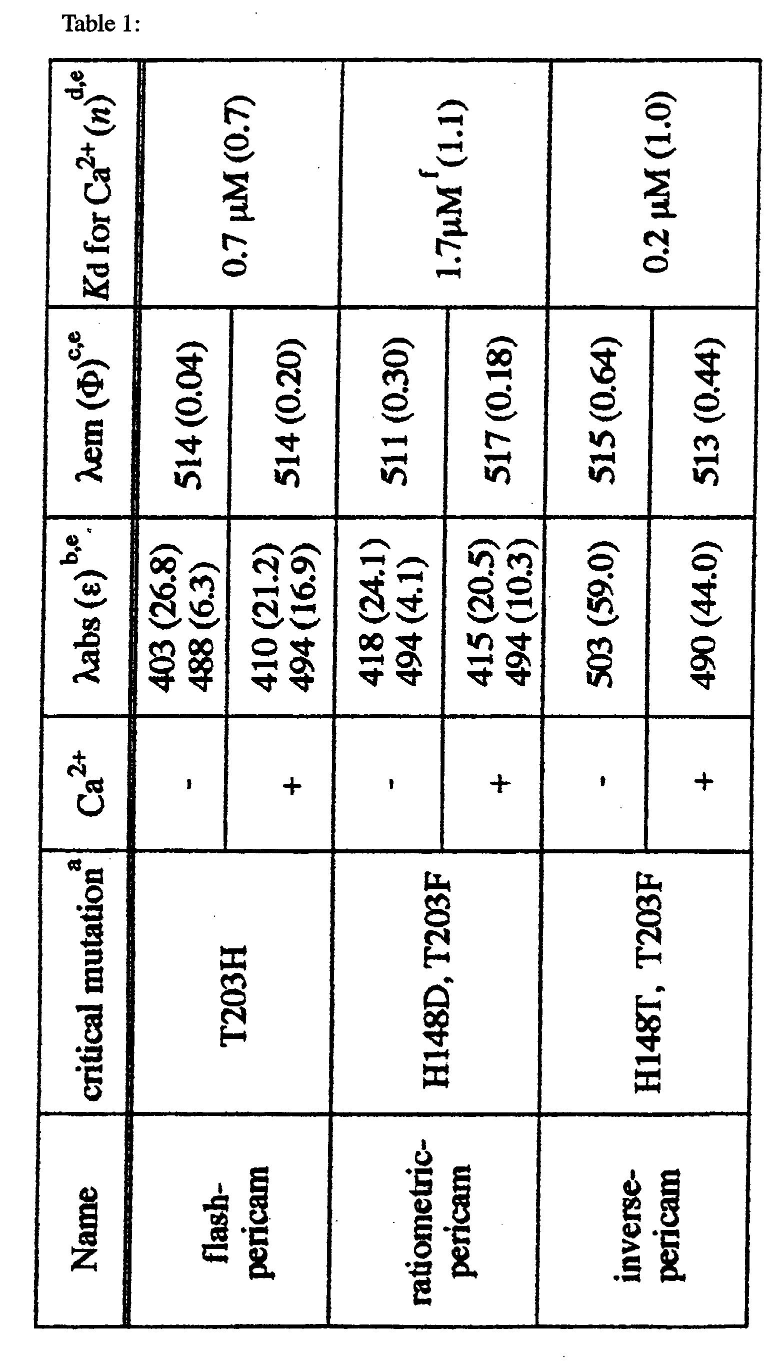

Table 1 shows spectral properties of flash-pericam, inverse-pericam, and ratiometric-pericam.
a: Substitutions from the primary sequence of EYFP(V68L/Q69K) are given as the single-letter code for the amino acid being replaced, its numerical position in the sequence, and the single-letter code for the replacement.
b: λ_{abs} is the peak of the absorbance spectrum in nanometer units, ε in parentheses is the absorbance extinction coefficient in 10³M⁻¹cm⁻¹.
c: λₑₘ is the peak of emission spectrum in nanometer units. Φ in parentheses is the fluorescence quantum yield.
d: Kd values for Ca²⁺ were estimated from the fitted curves in Figure 3, η in parentheses is the Hill coefficient.
e: These properties were measured in 50 mM HEPES/KOH pH7.4.
f: This value indicates *K*'d (apparent *K*d).

Most YFPs have a tyrosine or a histidine at position 203 and contain non-fluorescent protonated species absorbing at around 400 nm (Tsien, R Y. (1998) *Annu. Rev. Biochem.* 67, 509-44). Also, fluorescence was hardly detectable when flash-pericam was excited at around 400 nm. On the other hand, phenylalanine at position 203 in YFP was shown to make the protonated species fluorescent. For example, excitation of YFP with Phe203 at 400 nm gave rise to a predominant emission peak at 455 nm (Dickson, R M., Cubitt, A. B., Tsien, R Y. & Moerner, W. E. (1997) *Nature* 388, 355-358). Aiming for a dual-excitation ratiometric Ca²⁺ indicator, we introduced Phe203 in flash-pericam. Again, the linkers and several amino acids proved critical to the optimization of protein folding and the Ca²⁺ sensitivity. After numerous constructs had been tested, "ratiometric-pericam" was derived from flash-pericam by introducing H203F, H148D, F46L, deleting a glycine before CaM, and replacing the GGSGG linker with VDGGSGGTG, between the original N- and C-termini (Figure 1). As was seen in flash-pericam, Ca²⁺ binding promoted ionization of the chromophore in ratiometric-pericam. Therefoe, ratiometric-pericam exhibited a Ca²⁺-dependent change in the absorbance spectrum similar to flash-pericam (Figure 3B), and the pH-titration curve was shifted leftwards with Ca²⁺ (Figure 3H). In contrast to flash-pericam, however, ratiometric-pericam had a bimodal excitation spectrum peaking at 415 and 494 nm (Figure 3E), and the relative intensities of green fluorescence (511-517 nm) emitted when excited with 494- and 415-nm light was changed by about ten-fold between Ca²⁺-saturated and Ca²⁺-free forms (Figure 3E). The excitation ratio (494/415) showed a monophasic Ca²⁺-dependency with an apparent dissociation constant (*K*'d) of 1.7 µM and a Hill constant of 1.1 (Figure 3K).

During the semi-random mutagenesis on ratiometric-pericam, we found an interesting protein, which had a substitution D148T (Figure 1). In contrast to flash-pericam, the green fluorescence (513-515 nm) emitted when excited at 500 nm decreased to 15% with Ca²⁺ (Figure 3F). Thus, the protein has been named "inverse-pericam". The binding of Ca²⁺ to inverse-pericam may have promoted the protonation of the chromophore. However, the following results indicate this was not the case. At pH 7.4, Ca²⁺ ion only blue-shifted the peak of absorbance spectrum from 503 to 490 nm, with a tiny hump around 400 nm unaltered (Figure 3C); the Ca²⁺ binding did not affect the protonation state of the chromophore. Also, both the Ca²⁺-bound and Ca²⁺-free inverse-pericams were pH-titrated in a similar way, however, in the ionized states (pH>8), the Ca²⁺-free protein was seven-fold brighter than the Ca²⁺-bound form (Figure 3I). In fact, the quantum yield was decreased by 30% with Ca²⁺ binding (Table 1). These results suggest that the change in fluorescence intensity would be explained mostly by direct effects of the Ca²⁺-related structural change on the chromophore.

### (B-2) [Ca²⁺]ᵢ imaging in HeLa cells expressing pericams

When each pericam was transfected into HeLa cells, the fluorescence was uniformly distributed throughout the cytosolic and nuclear compartments but excluded from the nucleoli, as expected for a 44 kDa protein, which can pass through the nuclear pores (for example, see Figure 5A). Thus, the pericams can report intracellular free Ca²⁺ concentrations ([Ca²⁺]ᵢ). Figures 4A, 4B and 4C show receptor-stimulated [Ca²⁺]ᵢ oscillations in single HeLa cells expressing flash-pericam, ratiometric-pericam and inverse-pericam, respectively. In each case, the response began with a large spike, became sinusoidal, then converted into transient spikes and gradually declining frequency.

Compared with the single-wavelength indicators (flash-pericam and inverse-pericam), ratiometric-pericam permits quantitative Ca²⁺ imaging that can cancel out artifacts caused by indicator concentration and cell thickness or movement. In the presence of Ca²⁺ ionophore, the application of a high concentration (5 mM) of extracellular Ca²⁺, then BAPTA-AM and EGTA gave the maximum and minimum ratios (*R*ₘₐₓ and *R*ₘᵢₙ, respectively) for an *in situ* calibration (Figure 4B, top). While the amplitude of the first spike was about 3 µM, that of the subsequent spikes was below 1 µM. The relatively weak Ca²⁺ affinity allows for a more precise quantification of spike amplitudes than fura-2, the popular dual-excitation ratiometric Ca²⁺ indicator.

The time course of inverse-pericam (Figure 4C) seems to be a symmetric image of the flash-pericam (Figure 4A). However, inverse-pericam indicated relatively more sustained [Ca²⁺]ᵢ increases (Figure 4C). This is consistent with the finding that inverse-pericam had a higher affinity for Ca²⁺ than flash-pericam; their *K*d values were 0.2 and 0.7 µM, respectively (Figures 3J and 3L). One advantageous feature of inverse-pericam is that its Ca²⁺-free form has a high molar extinction coefficient and quantum yield at pH 7.4 (Table 1), which are almost comparable to those of enhanced GFP (Tsien, R Y. (1998) *Annu. Rev. Biochem*. 67, 509-44). Thus, despite the unusual characteristics of dimming with Ca²⁺, the use of inverse-pericam permits Ca²⁺ imaging with a satisfactory signal-to-noise ratio.

In the course of the improvement of pericams, folding efficiency at 37°C in mammalian cells was also an important factor. Inverse-pericam and ratiometric-pericam can be efficiently folded independently of temperature, while flash-pericam is better produced at 28-30°C.

### (B-3) Monitoring protein hereto-dimerization

An intriguing question is whether the circularly permuted GFP variants can detect intermolecular associations. To address this, we split flash-pericam at the linker between the original N- and C-termini of EYFP(V68L/Q69K) (Figure 1). The two proteins ("split-pericam") were co-expressed in HeLa cells. Although the chromophore of GFP is made of the tripeptide 65-67, its formation requires the entire β-can structure (Tsien, R Y. (1998) *Annu. Rev. Biochem.* 67, 509-44). It was not clear, therefore, how efficiently the two peptide chains associated at the basal level of [Ca²⁺]ᵢ, and how much the mature chromophore was formed. In spite of such uncertainty, split-pericam was able to monitor the reversible association between CaM and M13 peptide. The temporal profile almost reflected [Ca²⁺]ᵢ oscillations caused by ATP or histamine (Figure 4D). However, split-pericam could not be used as a reliable Ca²⁺ indicator: CaM and M13 peptide being separate, they are fully subject to cross-reaction with unlabelled CaM and CaM-binding proteins in cells. Our *in vitro* studies have revealed that the fused CaM and M13 in flash-pericam preferentially interact with each other.

### (B-4) Permeability of the nuclear envelope to Ca²⁺ ion

It has been controversial whether nuclear free Ca²⁺ concentration ([Ca²⁺]ₙ) is regulated independently cytosolic free Ca²⁺ concentration ([Ca²⁺]_{c}) (Malviya A. N. & Rogue, P. J. (1998) *Cell* 92, 17-23). Comparative measurements of [Ca²⁺]ₙ and [Ca²⁺]_{c} have been carried out using synthetic fluorescent chelators. However, the cytosolic signal is often contaminated by signals from intracellular organelles such as endoplasmic reticulum, in which the chelators can be compartmentalized (Brown, G R, Kohler, M & Berggren, P. O. (1997) *Biochem. J.* 325, 771-778). The Ca²⁺ sensitive photoprotein, aequorin has been localized specifically in nucleus and cytosol (Badminton, M. N., Campbell, A. K. & Rembold, C. M. (1996) *J. Biol. Chem.* 271, 31210-31214; and Brini, M., Murgia, M., Pasti, L., Picard, D., Pozzan, T. & Rizzuto, R. (1993) *EMBO J.* 12, 4813-4819), but it was proven difficult to image [Ca²⁺]ₙ and [Ca²⁺]_{c} in individual cells. To examine the propagation of Ca²⁺ waves, we transfected HeLa cells with flash-pericam cDNA and observed the signals using high-speed confocal line-scanning microscopy equipped with an argon-ion laser. Figure 5A shows an optically-sectioned image of a cell expressing flash-pericam, which was always distributed throughout the cytosol and nucleus at the same protein concentration as mentioned above. Thus, the simple use of flash-pericam allowed us to compare the two Ca²⁺ concentrations without concern on the difference in Ca²⁺ buffering. A typical pattern of the Ca²⁺ propagation stimulated by histamine and detected by line-scanning is shown in Figure 5B. The Ca²⁺ waves were traveled along the line indicated in Figure 5A. Figure 5C gives the time course measurement of [Ca²⁺]ₙ and [Ca²⁺]_{c} after bath-application of 1 µM histamine. No obvious difference in temporal profiles between [Ca²⁺]ₙ and [Ca²⁺]_{c} was evident; similar results were obtained from the experiments using video-rate scanning two-photon excitation fluorescence microscopy (Fan, G Y., Fujisaki, H., Miyawaki, A., Tsay, R. K., Tsien, R Y. & Ellisman, M. H. (1999) *Biophys. J.* 76, 2412-2420) and fluorescent Ca²⁺ indicator proteins, cameleons (Miyawaki, A., Llopis, J., Heim, R, McCaffery, J. M., Adams, J. A., Ikura, M. & Tsien, R Y. (1997) *Nature* 388, 882-887).

### (B-5) Free Ca²⁺ dynamics in mitochondria

Mitochondria are active participants in cellular Ca²⁺ signaling. Mitochondria sequester Ca²⁺ from cytosolic microdomains of high Ca²⁺ concentration generated by the entry or release of Ca²⁺ from intracellular stores. Although it is interesting to know how the [Ca²⁺]_{c} increases are relayed into mitochondria, only few studies have succeeded in simultaneous measurements of [Ca²⁺]_{c} and [Ca²⁺]ₘ (free Ca²⁺ concentration in mitochondria) in single intact cells (Ricken, S., Leipziger, J., Greger, R & Nitschke, R (1998) *J. Biol. Chem.* 273, 34961-34969). Synthetic fluorescent chelators are difficult to be differentially targeted. Thus the measurements have employed patch clamp procedures; for example, after accumulation of the permeant cation rhod-2 acetoxymethyl ester in mitochondria, other Ca²⁺ dyes conjugated with dextran were loaded by whole cell dialysis (Babcock, D., Herrington, J., Goodwin, P. C., Park, Y. B. & Hille, B. (1997) *J. Cell Biol.* 136, 833-844), where the mitochondria were not bathed in the natural cytoplasm. On the other hand, aequorin is easily targeted (Rutter, G A. Burnett, P., Rizzuto, R, Brini, M., Murgia, M., Pozzan, T., Tavare, J. M. & Denton, R. M. (1996) *Proc. Natl. Acad. Sci. USA* 93, 5489-5494) but requires the incorporation of coelenterazine, is irreversibly consumed by Ca²⁺, and is difficult to image because its luminescence produces less than one photon per molecule.

We measured [Ca²⁺]ₘ and [Ca²⁺]ₙ using ratiometric-pericams targeted to mitochondria and nucleus, respectively, in HeLa cells. Based on our finding that Ca²⁺ signals passed freely from the cytosol to nucleus in HeLa cells, we measured [Ca²⁺]ₙ as the extra-mitochondrial Ca²⁺ signal. This allows for simultaneous observations of intra- and extra-mitochondrial Ca²⁺ signals to be performed, which were well separated spatially. Figure 6A shows 4 snapshots of a HeLa cell 0, 5, 10, and 65 seconds after perfusion with 1 µM histamine. The excitation ratios (480/410) measured in the two spots indicated in the 0 s image are overlaid (Figure 6B). A sustained increase in [Ca²⁺]ₙ was evoked, which was accompanied by a transient and synchronized increase in [Ca²⁺]ₘ. However, the peak of [Ca²⁺]ₘ lagged behind [Ca²⁺]ₙ by 5 to 10 seconds as reported (Ricken, S., Leipziger, J., Greger, R & Nitschke, R (1998) *J. Biol. Chem.* 273, 34961-34969). Although previous studies which compared [Ca²⁺]ₘ and [Ca²⁺]ₙ in whole cell patch mode reported delayed decrease in [Ca²⁺]ₘ, the recovery of [Ca²⁺]ₘ was rather fast in our experiments, consistent with the results of another experiment using intact cells (Ricken, S., Leipziger, J., Greger, R & Nitschke, R (1998) *J. Biol. Chem.* 273, 34961-34969). It is therefore possible that the Ca²⁺ extrusion from mitochondria might be regulated by cytosolic factors which were lost in the whole cell dialysis. Figure 6A also shows that during the falling phase, some mitochondria transiently took up more Ca²⁺ than others as indicated by an arrow in the 65 s image. The local increases in [Ca²⁺]ₘ were reproducibly observed. In two HeLa cells with only mitochondrially-targeted ratiometric-pericam (Figure 6C), the single mitrochondria showing high [Ca²⁺]ₘ were clearly imaged (indicated by arrows). Each of the [Ca²⁺]ₘ rises appeared to last for about 10 seconds. Such different behaviors of mitochondrial populations in individual cells were revealed by a subcellular imaging of [Ca²⁺]ₘ using aequorin (Montero, M. Alonso, M. T., Carnicero, E., Cuchillo-Ibanez, I. Albillos, A., Garcia, A. G, Garcia-Sancho, J. & Alvarez., J. (2000) *Nat. Cell Biol.* 2, 57-61).

### (B-6) Comprison of pericams with other genetically-encodable Ca²⁺ indicators

Two genetically-encodable fluorescent Ca²⁺ indicators were previously reported: cameleon (Miyawaki, A., Llopis, J., Heim, R, McCaffery, J. M., Adams, J. A., Ikura, M. & Tsien, R Y. (1997) *Nature* 388, 882-887) and camgaroo (Baird, G S., Zacharias, D. A. & Tsien, R Y (1999) *Proc. Natl. Acad. Sci*. USA 96, 11241-11246). Cameleon is a dual emission ratiometric Ca²⁺ indicator that employs FRET with 2 GFP mutants of different color. Compared with cameleon, ratiometric-pericam shows greater responses to Ca²⁺. The ten-fold ratio of *R*ₘₐₓ to *R*ₘᵢₙ of ratiometric-pericam inside HeLa cells, which is consistent with the excitation ratio (494/415) change obtained *in vitro,* is much greater than the 2-fold ratio of *R*ₘₐₓ/*R*ₘᵢₙ of the improved yellow cameleon (Miyawaki, A., Griesbeck, O., Heim, R & Tsien, R Y. (1999) *Proc. Natl. Acad. Sci. USA* 96, 2135-2140). Ratiometric-pericam also guarantees a sufficient signal-to-noise ratio, since it shows relatively high absorbance extinction coefficient and quantum yield, and efficient folding ability at 37°C. In this example, mitochondrially-targeted ratiometric-pericam allowed us to monitor [Ca²⁺]ₘ in single mitochondria.

Camgaroo is a Ca²⁺-sensitive EYFP that has an insertion of calmodulin at a central site, and becomes 7-fold brighter upon saturation with Ca²⁺ like flash-pericam. However, due to its low affinity (*K*d = 7 µM) for Ca²⁺, camgaroo can not detect well the histamine-induced [Ca²⁺]ᵢ spikes of up to 2 to 3 µM. By contrast, flash-pericam has a reasonably high affinity (*K*d = 0.7 µM) for Ca²⁺, thereby capable of sensing physiological changes in [Ca²⁺]ᵢ.

By this example, we demonstrate that it is possible to change dramarically the Ca²⁺-dependent behavior of pericam just by introducing subtle mutations in the amino acids close to the chromophore. Although, in most cases, pericam was randomly mutated, there were some rational mutations. One example was the substitution of phenylalanine for tyrosine at residue 203 (Y203F), for creating ratiometric-pericam. Crystallographic studies on the three pericams would give us more information for designing pericams with different behaviors or other cpGFP-based biosensors. Through more profound understanding on the proton network of GFP, cpGFPs should potentially become powerful tools, complementing the FRET technology.

### Industrial Applicability

According to the present invention, it has become possible to provide a novel fluorescent protein having sensitivity to a calcium ion, in particular a fluorescent protein which can be used as an indicator for quantifying calcium ion concentration.

## Claims

1. A fluorescent protein having the following amino acid sequences (1) to (3) in order in the direction from the N-terminus to the C-terminus, wherein a fused fluorescent protein obtained by fusion of the fluorescent protein with a calcium binding protein and its target peptide can emit fluorescence which is dependent on Ca²⁺ ion level;
(1) an amino acid sequence from the n^{th} amino acid from the N-terminus to the C-terminus of a fluorescent protein selected from the group consisting of a green fluorescent protein or its mutant, a yellow fluorescent protein or its mutant, a cyan fluorescent protein or its mutant, a red fluorescent protein or its mutant, and a blue fluorescent protein or its mutant, provided that n represents an integer of 140 to 150;
(2) a linker sequence of a sequence of 2 to 20 amino acids; and
(3) an amino acid sequence from the 1^{st} amino acid to the (n-1)^{th} amino acid from the N-terminus of the fluorescent protein described in (1) above.

2. The fluorescent protein according to claim 1 wherein, under the presence of Ca²⁺ ions, the fused fluorescent protein can emit fluorescence of an intensity which differs depending on Ca²⁺ ion level.

3. The fluorescent protein according to claim 1 wherein, under the presence of Ca²⁺ ions, the fused fluorescent protein can show excitation of a wavelength which differs depending on Ca²⁺ ion level.

4. The fluorescent protein according to claim 1 wherein the amino acid sequence of a linker sequence is Gly-Gly-Ser-Gly-Gly or Val-Asp-Gly-Gly-Ser-Gly-Gly-Thr-Gly.

5. A fluorescent protein of any of the followings;
(A) a protein having the amino acids from position 32 to 275 of the amino acid sequence shown in SEQ ID NO:1, or a protein having an amino acid sequence having a deletion, substitution and/or addition of one to several amino acids in the amino acids from position 32 to 275 of the amino acid sequence shown in SEQ ID NO:1 and having a fluorescence characteristic that is equivalent to or greater than that of a protein having the amino acids from position 32 to 275 of the amino acid sequence shown in SEQ ID NO:1;
(B) a protein having the amino acids from position 32 to 278 of the amino acid sequence shown in SEQ ID NO:2, or a protein having an amino acid sequence having a deletion, substitution and/or addition of one to several amino acids in the amino acids from position 32 to 278 of the amino acid sequence shown in SEQ ID NO:2 and having a fluorescence characteristic that is equivalent to or greater than that of a protein having the amino acids from position 32 to 278 of the amino acid sequence shown in SEQ ID NO:2; or,
(C) a protein having the amino acids from position 32 to 278 of the amino acid sequence shown in SEQ ID NO:3, or a protein having an amino acid sequence having a deletion, substitution and/or addition of one to several amino acids in the amino acids from position 32 to 278 of the amino acid sequence shown in SEQ ID NO:3 and having a fluorescence characteristic that is equivalent to or greater than that of a protein having the amino acids from position 32 to 278 of the amino acid sequence shown in SEQ ID NO:3.

6. A fused fluorescent protein having the following amino acid sequences (1) to (5) in order in the direction from the N-terminus to the C-terminus, which can emit fluorescence that is dependent on Ca²⁺ ion level.
(1) an amino acid sequence of a target peptide of a calcium-binding protein;
(2) an amino acid sequence from the n^{th} amino acid from the N-terminus to the C-terminus of a fluorescent protein selected from the group consisting of a green fluorescent protein or its mutant, a yellow fluorescent protein or its mutant, a cyan fluorescent protein or its mutant, a red fluorescent protein or its mutant, and a blue fluorescent protein or its mutant, provided that n represents an integer of 140 to 150;
(3) a linker sequence of a sequence of 2 to 20 amino acids;
(4) an amino acid sequence from the 1^{st} amino acid to the (n-1)^{th} amino acid from the N-terminus of the fluorescent protein described in (2) above; and
(5) the amino acid sequence of a calcium-binding protein.

7. The fused fluorescent protein according to claim 6 which can, under the presence of Ca²⁺ ions, emit fluorescence of an intensity that differs depending on Ca²⁺ ion level.

8. The fused fluorescent protein according to claim 6 which can, under the presence of Ca²⁺ ions, show excitation of a wavelength that differs depending on Ca²⁺ ion level.

9. The fused fluorescent protein according to claim 6 wherein the amino acid sequence of a linker sequence is Gly-Gly-Ser-Gly-Gly or Val-Asp-Gly-Gly-Ser-Gly-Gly-Thr-Gly.

10. The fused fluorescent protein according to claim 6 wherein the calcium-binding protein is a protein selected from the group consisting of: calmodulin, troponin C, calcineurin B, myosin light chain, recoverin, S-modulin, visinin, VILIP, neurocalcin, hippocalcin, frequenin, caltractin, calpain large-subunit, S100 proteins, parvalbumin, calbindin D_{9K}, calbindin D_{28K}, and calretinin.

11. A fused fluorescent protein of any of the followings;
(A) a protein having the amino acid sequence shown in SEQ ID NO:1, or a protein having an amino acid sequence having a deletion, substitution and/or addition of one to several amino acids in the amino acid sequence shown in SEQ ID NO:1 and having a fluorescence characteristic that is equivalent to or greater than that of a protein having the amino acid sequence shown in SEQ ID NO:1;
(B) a protein having the amino acid sequence shown in SEQ ID NO:2, or a protein having an amino acid sequence having a deletion, substitution and/or addition of one to several amino acids in the amino acid sequence shown in SEQ ID NO:2 and having a fluorescence characteristic that is equivalent to or greater than that of a protein having the amino acid sequence shown in SEQ ID NO:2; or,
(C) a protein having the amino acid sequence shown in SEQ ID NO:3, or a protein having an amino acid sequence having a deletion, substitution and/or addition of one to several amino acids in the amino acid sequence shown in SEQ ID NO:3 and having a fluorescence characteristic that is equivalent to or greater than that of a protein having the amino acid sequence shown in SEQ ID NO:3.

12. A calcium ion indicator which comprises the fused fluorescent protein of claim 6.

13. A method for measuring concentration or distribution of intracellular calcium ion by using the fused fluorescent protein of claim 6.

14. DNA encoding the fluorescent protein of claim 1.

15. DNA of any of the followings;
(A) DNA having the nucleotide sequence from position 94 to 825 of the nucleotide sequence shown in SEQ ID NO:1, or DNA having a nucleotide sequence having a deletion, substitution and/or addition of one to several nucleotides in the nucleotide sequence from position 94 to 825 of the nucleotide sequence shown in SEQ ID NO:1 and encoding a protein having a fluorescence characteristic that is equivalent to or greater than that of a protein encoded by DNA having the nucleotide sequence from position 94 to 825 of the nucleotide sequence shown in SEQ ID NO:1;
(B) DNA having the nucleotide sequence from position 94 to 834 of the nucleotide sequence shown in SEQ ID NO:2, or DNA having a nucleotide sequence having a deletion, substitution and/or addition of one to several nucleotides in the nucleotide sequence from position 94 to 834 of the nucleotide sequence shown in SEQ ID NO:2 and encoding a protein having a fluorescence characteristic that is equivalent to or greater than that of a protein encoded by DNA having the nucleotide sequence from position 94 to 834 of the nucleotide sequence shown in SEQ ID NO:2; or,
(C) DNA having the nucleotide sequence from position 94 to 834 of the nucleotide sequence shown in SEQ ID NO:3, or DNA having a nucleotide sequence having a deletion, substitution and/or addition of one to several nucleotides in the nucleotide sequence from position 94 to 834 of the nucleotide sequence shown in SEQ ID NO:3 and encoding a protein having a fluorescence characteristic that is equivalent to or greater than that of a protein encoded by DNA having the nucleotide sequence from position 94 to 834 of the nucleotide sequence shown in SEQ ID NO:3.

16. DNA encoding the fused fluorescent protein of claim 6.

17. DNA of any of the followings:
(A) DNA having the nucleotide sequence shown in SEQ ID NO:1, or DNA having a nucleotide sequence having a deletion, substitution and/or addition of one to several nucleotides in the nucleotide sequence shown in SEQ ID NO:1 and encoding a protein having a fluorescence characteristic that is equivalent to or greater than that of a protein encoded by the nucleotide sequence shown in SEQ ID NO:1;
(B) DNA having the nucleotide sequence shown in SEQ ID NO:2, or DNA having a nucleotide sequence having a deletion, substitution and/or addition of one to several nucleotides in the nucleotide sequence shown in SEQ ID NO:2 and encoding a protein having a fluorescence characteristic that is equivalent to or greater than that of a protein encoded by the nucleotide sequence shown in SEQ ID NO:2; or
(C) DNA having the nucleotide sequence shown in SEQ ID NO:3, or DNA having a nucleotide sequence having a deletion, substitution and/or addition of one to several nucleotides in the nucleotide sequence shown in SEQ ID NO:3 and encoding a protein having a fluorescence characteristic that is equivalent to or greater than that of a protein encoded by the nucleotide sequence shown in SEQ ID NO:3.

18. A recombinant vector having the DNA of claim 14.

19. A transformant having the DNA of claim 14 or the recombinant vector of claim 18.

20. A method for measuring the calcium ion concentration or distribution in the transformant of claims 19 by using the fluorescence emitted by the transformant as an index.

21. A kit for measuring calcium ions which comprises at least one or more selected from the fluorescent protein of claim 1, the fused fluorescent protein of claim 6, the calcium ion indicator of claims 12, the DNA of claim 14, the recombinant vector of claim 18, or the transformant of claim 19.


SEQUENCE LISTING
<110> RIKEN
<120> Fluorescent proteins
<130> FA2103A/US
<160> 5

<210> 1
<211> 1278
<212> DNA
<213> Artificial Sequence
<220>
<223> Description of Artificial Sequence: Recombinant DNA
<400> 1
atg aag agg cgc tgg aag aaa aac ttc att gcc gtc agc gct gcc aac 48
Met Lys Arg Arg Trp Lys Lys Asn Phe Ile Ala Val Ser Ala Ala Asn
1               5                   10                  15
cgg ttc aag aag atc tcc agc tcc ggg gca ctg ggg tct gca ggc tac 96
Arg Phe Lys Lys Ile Ser Ser Ser Gly Ala Leu Gly Ser Ala Gly Tyr
            20                  25                  30
aac agc cac aac gtc tat atc atg gcc gac aag cag aag aac ggc atc 144
Asn Ser His Asn Val Tyr Ile Met Ala Asp Lys Gln Lys Asn Gly Ile
        35                  40                  45
aag gcc aac ttc aag atc cgc cac aac atc gag gac ggc ggc gtg cag 192
Lys Ala Asn Phe Lys Ile Arg His Asn Ile Glu Asp Gly Gly Val Gln
    50                  55                  60
ctc gcc gac cac tac cag cag aac acc ccc atc ggc gac ggc ccc gtg 240
Leu Ala Asp His Tyr Gln Gln Asn Thr Pro Ile Gly Asp Gly Pro Val
65                  70                  75                  80
ctg ctg ccc gac aac cac tac ctg agc cac cag tcc gcc ctg agc aaa 288

Leu Leu Pro Asp Asn His Tyr Leu Ser His Gln Ser Ala Leu Ser Lys
                85                  90                  95
gac ccc aac gag aag cgc gat cac atg gtc ctg ctg gag ttc gtg acc 336
Asp Pro Asn Glu Lys Arg Asp His Met Val Leu Leu Glu Phe Val Thr
            100                 105                 110
gcc gcc ggg atc act ctc ggc atg gac gag ctg tac aag ggt ggc agc 384
Ala Ala Gly Ile Thr Leu Gly Met Asp Glu Leu Tyr Lys Gly Gly Ser
        115                 120                 125
ggt ggc atg gtg agc aag ggc gag gag ctg ttc acc ggg gtg gtg ccc 432
Gly Gly Met Val Ser Lys Gly Glu Glu Leu Phe Thr Gly Val Val Pro
    130                 135                 140
atc ctg gtc gag ctg gac ggc gac gta aac ggc cac aag ttc agc gtg 480
Ile Leu Val Glu Leu Asp Gly Asp Val Asn Gly His Lys Phe Ser Val
145                 150                 155                 160
tcc ggc gag ggc gag ggc gat gcc acc tac ggc aag ctg acc ctg aag 528
Ser Gly Glu Gly Glu Gly Asp Ala Thr Tyr Gly Lys Leu Thr Leu Lys
                165                 170                 175
ttc atc tgc acc acc ggc aag ctg ccc gtg ccc tgg ccc acc ctc gtg 576
Phe Ile Cys Thr Thr Gly Lys Leu Pro Val Pro Trp Pro Thr Leu Val
            180                 185                 190
acc acc ttc ggc tac ggc ctg aag tgc ttc gcc cgc tac ccc gac cac 624
Thr Thr Phe Gly Tyr Gly Leu Lys Cys Phe Ala Arg Tyr Pro Asp His
        195                 200                 205
atg aag cag cac gac ttc ttc aag tcc gcc atg ccc gaa ggc tac gtc 672
Met Lys Gln His Asp Phe Phe Lys Ser Ala Met Pro Glu Gly Tyr Val
    210                 215                 220
cag gag cgc acc atc ttc ttc aag gac gac ggc aac tac aag acc cgc 720
Gln Glu Arg Thr Ile Phe Phe Lys Asp Asp Gly Asn Tyr Lys Thr Arg
225                 230                 235                 240

gcc gag gtg aag ttc gag ggc gac acc ctg gtg aac cgc atc gag ctg 768
Ala Glu Val Lys Phe Glu Gly Asp Thr Leu Val Asn Arg Ile Glu Leu
                245                 250                 255
aag ggc atc gac ttc aag gag gac ggc aac atc ctg ggg cac aag ctg 816
Lys Gly Ile Asp Phe Lys Glu Asp Gly Asn Ile Leu Gly His Lys Leu
            260                 265                 270
gag tac aac ggt acc ggg gac caa ctg aca gaa gag cag att gca gag 864
Glu Tyr Asn Gly Thr Gly Asp Gln Leu Thr Glu Glu Gln Ile Ala Glu
        275                 280                 285
ttc aaa gaa gcc ttc tca tta ttc gac aag gat ggg gac ggc acc atc 912
Phe Lys Glu Ala Phe Ser Leu Phe Asp Lys Asp Gly Asp Gly Thr Ile
    290                 295                 300
acc aca aag gaa ctt ggc acc gtt atg agg tcg ctt gga caa aac cca 960
Thr Thr Lys Glu Leu Gly Thr Val Met Arg Ser Leu Gly Gln Asn Pro
305                 310                 315                 320
acg gaa gca gaa ttg cag gat atg atc aat gaa gtc gat gct gat ggc 1008
Thr Glu Ala Glu Leu Gln Asp Met Ile Asn Glu Val Asp Ala Asp Gly
                325                 330                 335
aat gga acg att tac ttt cct gaa ttt ctt act atg atg gct aga aaa 1056
Asn Gly Thr Ile Tyr Phe Pro Glu Phe Leu Thr Met Met Ala Arg Lys
            340                 345                 350
atg aag gac aca gac agc gaa gag gaa atc cga gaa gca ttc cgt gtt 1104
Met Lys Asp Thr Asp Ser Glu Glu Glu Ile Arg Glu Ala Phe Arg Val
        355                 360                 365
ttt gac aag gat ggg aac ggc tac atc agc gct gct cag tta cgt cac 1152
Phe Asp Lys Asp Gly Asn Gly Tyr Ile Ser Ala Ala Gln Leu Arg His
    370                 375                 380
gtc atg aca aac ctc ggg gag aag tta aca gat gaa gaa gtt gat gaa 1200
Val Met Thr Asn Leu Gly Glu Lys Leu Thr Asp Glu Glu Val Asp Glu

385                 390                 395                 400
atg ata agg gaa gca gat atc gat ggt gat ggc caa gta aac tat gaa 1248
Met Ile Arg Glu Ala Asp Ile Asp Gly Asp Gly Gln Val Asn Tyr Glu
                405                 410                 415
gag ttt gta caa atg atg aca gca aag taa 1278
Glu Phe Val Gln Met Met Thr Ala Lys
            420                 425

<210> 2
<211> 1284
<212> DNA
<213> Artificial Sequence
<220>
<223> Description of Artificial Sequence: Recombinant DNA
<400> 2
atg aag agg cgc tgg aag aaa aac ttc att gcc gtc agc gct gcc aac 48
Met Lys Arg Arg Trp Lys Lys Asn Phe Ile Ala Val Ser Ala Ala Asn
1               5                   10                  15
cgg ttc aag aag atc tcc agc tcc ggg gca ctg ggg tct gca ggc tac 96
Arg Phe Lys Lys Ile Ser Ser Ser Gly Ala Leu Gly Ser Ala Gly Tyr
            20                  25                  30
aac agc gac aac gtc tat atc atg gcc gac aag cag aag aac ggc atc 144
Asn Ser Asp Asn Val Tyr Ile Met Ala Asp Lys Gln Lys Asn Gly Ile
        35                  40                  45
aag gcc aac ttc aag atc cgc cac aac atc gag gac ggc ggc gtg cag 192
Lys Ala Asn Phe Lys Ile Arg His Asn Ile Glu Asp Gly Gly Val Gln
    50                  55                  60
ctc gcc gac cac tac cag cag aac acc ccc atc ggc gac ggc ccc gtg 240
Leu Ala Asp His Tyr Gln Gln Asn Thr Pro Ile Gly Asp Gly Pro Val

65                    70                    75                    80

ctg ctg ccc gac aac cac tac ctg agc ttc cag tcc gcc ctg agc aaa   288

Leu Leu Pro Asp Asn His Tyr Leu Ser Phe Gln Ser Ala Leu Ser Lys

                85                    90                    95

gac ccc aac gag aag cgc gat cac atg gtc ctg ctg gag ttc gtg acc   336

Asp Pro Asn Glu Lys Arg Asp His Met Val Leu Leu Glu Phe Val Thr

            100                   105                   110

gcc gcc ggg atc act ctc ggc atg gac gag ctg tac aag gtc gac ggt   384

Ala Ala Gly Ile Thr Leu Gly Met Asp Glu Leu Tyr Lys Val Asp Gly

        115                   120                   125

ggc agc ggt ggc acc ggt gtg agc aag ggc gag gag ctg ttc acc ggg   432

Gly Ser Gly Gly Thr Gly Val Ser Lys Gly Glu Glu Leu Phe Thr Gly

    130                   135                   140

gtg gtg ccc atc ctg gtc gag ctg gac ggc gac gta aac ggc cac aag   480

Val Val Pro Ile Leu Val Glu Leu Asp Gly Asp Val Asn Gly His Lys

145                   150                   155                   160

ttc agc gtg tcc ggc gag ggc gag ggc gat gcc acc tac ggc aag ctg   528

Phe Ser Val Ser Gly Glu Gly Glu Gly Asp Ala Thr Tyr Gly Lys Leu

                165                   170                   175

acc ctg aag ctc atc tgc acc acc ggc aag ctg ccc gtg ccc tgg ccc   576

Thr Leu Lys Leu Ile Cys Thr Thr Gly Lys Leu Pro Val Pro Trp Pro

            180                   185                   190

acc ctc gtg acc acc ttc ggc tac ggc ctg aag tgc ttc gcc cgc tac   624

Thr Leu Val Thr Thr Phe Gly Tyr Gly Leu Lys Cys Phe Ala Arg Tyr

        195                   200                   205

ccc gac cac atg aag cag cac gac ttc ttc aag tcc gcc atg ccc gaa   672

Pro Asp His Met Lys Gln His Asp Phe Phe Lys Ser Ala Met Pro Glu

    210                   215                   220

ggc tac gtc cag gag cgc acc atc ttc ttc aag gac gac ggc aac tac   720

Gly Tyr Val Gln Glu Arg Thr Ile Phe Phe Lys Asp Asp Gly Asn Tyr
225                 230                 235                 240
aag acc cgc gcc gag gtg aag ttc gag ggc gac acc ctg gtg aac cgc 768
Lys Thr Arg Ala Glu Val Lys Phe Glu Gly Asp Thr Leu Val Asn Arg
                245                 250                 255
atc gag ctg aag ggc atc gac ttc aag gag gac ggc aac atc ctg ggg 816
Ile Glu Leu Lys Gly Ile Asp Phe Lys Glu Asp Gly Asn Ile Leu Gly
            260                 265                 270
cac aag ctg gag tac aac ggt acc gac caa ctg aca gaa gag cag att 864
His Lys Leu Glu Tyr Asn Gly Thr Asp Gln Leu Thr Glu Glu Gln Ile
        275                 280                 285
gca gag ttc aaa gaa gcc ttc tca tta ttc gac aag gat ggg gac ggc 912
Ala Glu Phe Lys Glu Ala Phe Ser Leu Phe Asp Lys Asp Gly Asp Gly
    290                 295                 300
acc atc acc aca aag gaa ctt ggc acc gtt atg agg tcg ctt gga caa 960
Thr Ile Thr Thr Lys Glu Leu Gly Thr Val Met Arg Ser Leu Gly Gln
305                 310                 315                 320
aac cca acg gaa gca gaa ttg cag gat atg atc aat gaa gtc gat gct 1008
Asn Pro Thr Glu Ala Glu Leu Gln Asp Met Ile Asn Glu Val Asp Ala
                325                 330                 335
gat ggc aat gga Acg att tac ttt cct gaa ttt ctt act atg atg gct 1056
Asp Gly Asn Gly Thr Ile Tyr Phe Pro Glu Phe Leu Thr Met Met Ala
            340                 345                 350
aga aaa atg aag gac aca gac agc gaa gag gaa atc cga gaa gca ttc 1104
Arg Lys Met Lys Asp Thr Asp Ser Glu Glu Glu Ile Arg Glu Ala Phe
        355                 360                 365
cgt gtt ttt gac aag gat ggg aac ggc tac atc agc gct gct cag tta 1152
Arg Val Phe Asp Lys Asp Gly Asn Gly Tyr Ile Ser Ala Ala Gln Leu
    370                 375                 380

cgt cac gtc atg aca aac ctc ggg gag aag tta aca gat gaa gaa gtt 1200
Arg His Val Met Thr Asn Leu Gly Glu Lys Leu Thr Asp Glu Glu Val
385                 390                 395                 400
gat gaa atg ata agg gaa gca gat atc gat ggt gat ggc caa gta aac 1248
Asp Glu Met Ile Arg Glu Ala Asp Ile Asp Gly Asp Gly Gln Val Asn
                405                 410                 415
tat gaa gag ttt gta caa atg atg aca gca aag taa 1284
Tyr Glu Glu Phe Val Gln Met Met Thr Ala Lys
            420                 425

<210> 3
<211> 1284
<212> DNA
<213> Artificial Sequence
<220>
<223> Description of Artificial Sequence: Recombinant DNA
<400> 3
atg aag agg cgc tgg aag aaa aac ttc att gcc gtc agc gct gcc aac 48
Met Lys Arg Arg Trp Lys Lys Asn Phe Ile Ala Val Ser Ala Ala Asn
1               5                   10                  15
cgg ttc aag aag atc tcc agc tcc ggg gca ctg ggg tct gca ggc tac 96
Arg Phe Lys Lys Ile Ser Ser Ser Gly Ala Leu Gly Ser Ala Gly Tyr
            20                  25                  30
aac agc gac aac gtc tat atc atg gcc gac aag cag aag aac ggc atc 144
Asn Ser Asp Asn Val Tyr Ile Met Ala Asp Lys Gln Lys Asn Gly Ile
        35                  40                  45
aag gcc aac ttc aag atc cgc cac aac atc gag gac ggc ggc gtg cag 192
Lys Ala Asn Phe Lys Ile Arg His Asn Ile Glu Asp Gly Gly Val Gln
    50                  55                  60

ctc gcc gac cac tac cag cag aac acc ccc atc ggc gac ggc ccc gtg 240
Leu Ala Asp His Tyr Gln Gln Asn Thr Pro Ile Gly Asp Gly Pro Val
65                  70                  75                  80
ctg ctg ccc gac aac cac tac ctg agc ttc cag tcc gcc ctg agc aaa 288
Leu Leu Pro Asp Asn His Tyr Leu Ser Phe Gln Ser Ala Leu Ser Lys
                85                  90                  95
gac ccc aac gag aag cgc gat cac atg gtc ctg ctg gag ttc gtg acc 336
Asp Pro Asn Glu Lys Arg Asp His Met Val Leu Leu Glu Phe Val Thr
            100                 105                 110
gcc gcc ggg atc act ctc ggc atg gac gag ctg tac aag gtc gac ggt 384
Ala Ala Gly Ile Thr Leu Gly Met Asp Glu Leu Tyr Lys Val Asp Gly
        115                 120                 125
ggc agc ggt ggc acc ggt gtg agc aag ggc gag gag ctg ttc acc ggg 432
Gly Ser Gly Gly Thr Gly Val Ser Lys Gly Glu Glu Leu Phe Thr Gly
    130                 135                 140
gtg gtg ccc atc ctg gtc gag ctg gac ggc gac gta aac ggc cac aag 480
Val Val Pro Ile Leu Val Glu Leu Asp Gly Asp Val Asn Gly His Lys
145                 150                 155                 160
ttc agc gtg tcc ggc gag ggc gag ggc gat gcc acc tac ggc aag ctg 528
Phe Ser Val Ser Gly Glu Gly Glu Gly Asp Ala Thr Tyr Gly Lys Leu
                165                 170                 175
acc ctg aag ctc atc tgc acc acc ggc aag ctg ccc gtg ccc tgg ccc 576
Thr Leu Lys Leu Ile Cys Thr Thr Gly Lys Leu Pro Val Pro Trp Pro
            180                 185                 190
acc ctc gtg acc acc ttc ggc tac ggc ctg aag tgc ttc gcc cgc tac 624
Thr Leu Val Thr Thr Phe Gly Tyr Gly Leu Lys Cys Phe Ala Arg Tyr
        195                 200                 205
ccc gac cac atg aag cag cac gac ttc ttc aag tcc gcc atg ccc gaa 672
Pro Asp His Met Lys Gln His Asp Phe Phe Lys Ser Ala Met Pro Glu

    210                 215                 220

ggc tac gtc cag gag cgc acc atc ttc ttc aag gac gac ggc aac tac 720

Gly Tyr Val Gln Glu Arg Thr Ile Phe Phe Lys Asp Asp Gly Asn Tyr

225                 230                 235                 240

aag acc cgc gcc gag gtg aag ttc gag ggc gac acc ctg gtg aac cgc 768

Lys Thr Arg Ala Glu Val Lys Phe Glu Gly Asp Thr Leu Val Asn Arg

                245                 250                 255

atc gag ctg aag ggc atc gac ttc aag gag gac ggc aac atc ctg ggg 816

Ile Glu Leu Lys Gly Ile Asp Phe Lys Glu Asp Gly Asn Ile Leu Gly

            260                 265                 270

cac aag ctg gag tac aac ggt acc gac caa ctg aca gaa gag cag att 864

His Lys Leu Glu Tyr Asn Gly Thr Asp Gln Leu Thr Glu Glu Gln Ile

        275                 280                 285

gca gag ttc aaa gaa gcc ttc tca tta ttc gac aag gat ggg gac ggc 912

Ala Glu Phe Lys Glu Ala Phe Ser Leu Phe Asp Lys Asp Gly Asp Gly

    290                 295                 300

acc atc acc aca aag gaa ctt ggc acc gtt atg agg tcg ctt gga caa 960

Thr Ile Thr Thr Lys Glu Leu Gly Thr Val Met Arg Ser Leu Gly Gln

305                 310                 315                 320

aac cca acg gaa gca gaa ttg cag gat atg atc aat gaa gtc gat gct 1008

Asn Pro Thr Glu Ala Glu Leu Gln Asp Met Ile Asn Glu Val Asp Ala

                325                 330                 335

gat ggc aat gga acg att tac ttt cct gaa ttt ctt act atg atg gct 1056

Asp Gly Asn Gly Thr Ile Tyr Phe Pro Glu Phe Leu Thr Met Met Ala

            340                 345                 350

aga aaa atg aag gac aca gac agc gaa gag gaa atc cga gaa gca ttc 1104

Arg Lys Met Lys Asp Thr Asp Ser Glu Glu Glu Ile Arg Glu Ala Phe

        355                 360                 365

cgt gtt ttt gac aag gat ggg aac ggc tac atc agc gct gct cag tta 1152

Arg Val Phe Asp Lys Asp Gly Asn Gly Tyr Ile Ser Ala Ala Gln Leu
    370                 375                 380
cgt cac gtc atg aca aac ctc ggg gag aag tta aca gat gaa gaa gtt 1200
Arg His Val Met Thr Asn Leu Gly Glu Lys Leu Thr Asp Glu Glu Val
385                 390                 395                 400
gat gaa atg ata agg gaa gca gat atc gat ggt gat ggc caa gta aac 1248
Asp Glu Met Ile Arg Glu Ala Asp Ile Asp Gly Asp Gly Gln Val Asn
                405                 410                 415
tat gaa gag ttt gta caa atg atg aca gca aag taa 1284
Tyr Glu Glu Phe Val Gln Met Met Thr Ala Lys
            420                 425

<210> 4
<211> 5
<212> PRT
<213> Artificial Sequence
<220>
<223> Description of Artificial Sequence: linker peptide
<400> 4
Gly Gly Ser Gly Gly
1               5

<210> 5
<211> 9
<212> PRT
<213> Artificial Sequence
<220>
<223> Description of Artificial Sequence: linker peptide
<400> 5

Val Asp Gly Gly Ser Gly Gly Thr Gly
1               5